# EUROPEAN PATENT SPECIFICATION

(11) **EP 4 159 899 B1**
(45) Date of publication and mention of the grant of the patent: **08.07.2026**
(21) Application number: 22194336.8
(22) Date of filing: 07.09.2022
(51) Int. Cl.: D01D 13/02, D01D 4/00, B65H 63/00, B65H 67/048, D01D 5/08, D01D 4/02, G01N 33/36

(54) **YARN SPINNING SYSTEM AND SPUN YARN WINDING SYSTEM**
GARNSPINNVORRICHTUNG UND SPINNGARNWICKELSYSTEM
SYSTÈME DE FILATURE DE FIL ET SYSTÈME D'ENROULEMENT DE FIL FILÉ

(30) Priority: 01.10.2021 JP 2021162853
(43) Date of publication of application: 05.04.2023
(73) Proprietor: TMT Machinery, Inc., Osaka-shi, Osaka 541-0041 (JP)
(72) Inventor: NANAYAMA, Daisuke, Kyoto-shi, Kyoto, 612-8686 (JP)
(74) Representative: Hoffmann Eitle

(56) References cited:
- EP-A2- 0 580 071
- US-A- 5 661 880

## Description

### BACKGROUND OF THE INVENTION

The present invention relates to a yarn spinning system configured to spin out a yarn, and a spun yarn winding system configured to wind the yarn spun out from the yarn spinning system.

Japanese Laid-Open Patent Publication No. 2015-81400 discloses a spun yarn winding system which includes (i) a yarn spinning system including a melt spinning device (spinning apparatus) and (ii) a spun yarn take-up machine (spun yarn take-up winder). The spinning apparatus is configured to discharge a material (molten polymer) of a yarn (i.e., is configured to spin out a yarn) through a spinneret. The material is supplied to a spinning pack. The spun yarn take-up winder is configured to produce a package by winding the yarn spun out from the spinning apparatus.

Other examples of the prior art can be seen in documents EP 0 580 071 A2 and US 5 661 880 A.

### SUMMARY OF THE INVENTION

The quality of a yarn (e.g., dispersion of thickness of a yarn) spun out from a spinning apparatus may vary depending on various factors such as the temperature of a spinneret. Especially, immediately after the yarn starts to be spun out from the spinning apparatus, the temperature of the spinneret, etc. is not stable. Therefore, the quality of the yarn is also unstable. It takes some time for the quality of the yarn to become stable after the yarn starts to be spun out from the spinning apparatus. A yarn spun out before its quality becomes stable is typically worse in quality than a yarn spun out after its quality becomes stable. Therefore, these yarns need to be handled differently from each other.

Traditionally, the quality of a yarn which starts to be spun out from a spinning apparatus is visually determined by an operator, etc. or is managed based on whether a predetermined time elapses after the yarn starts to be spun out. However, when the determination of whether the quality of the yarn is stable (hereinafter, this determination will be referred to as quality determination) is performed by the operator, the burden on the operator is disadvantageously high. Meanwhile, when the management based on the elapse of time is performed, the following problems may occur. That is, a time required from the start of yarn spinning from the spinning apparatus to the stabilization of the quality of the yarn may vary depending on the brand of the yarn. It is therefore necessary to manage or set the above-described predetermined time of each brand, with the result that the burden on the operator may be increased. Alternatively, when the above-described predetermined time elapses before the quality of the yarn actually becomes stable, it may be incorrectly determined that the quality of the yarn becomes stable.

An object of the present invention is to determine whether the quality of a yarn is stable, without needing human workforce.

According to a first aspect of the invention, a yarn spinning system in accordance with claim 1 includes a spinning apparatus configured to spin out at least one yarn, and the spinning apparatus includes: at least one spinning pack including a spinneret configured to discharge molten polymer which is a material of the at least one yarn; and an information detection unit arranged to be able to detect information regarding the quality of the at least one yarn spun out from the spinneret. In this aspect, the yarn spinning system further includes a first control unit. This first control unit is programmed to: acquire at least one predetermined determination parameter based on a detection result of the information detection unit; and perform quality determination of whether the quality of the at least one yarn is stable, with use of the at least one determination parameter.

According to this aspect, the control unit is able to perform the quality determination with use of the detection result of the information detection unit. It is therefore possible to determine whether the quality of the at least one yarn is stable, without needing human workforce.

According to the first aspect of the invention, the yarn spinning system is arranged such that the information detection unit includes a pressure detection unit which is configured to detect pressure of the molten polymer flowing into the at least one spinning pack. Optionally, the information detection unit may further include a temperature detection unit which is configured to detect a surface temperature of the spinneret.

The pressure of the molten polymer flowing into the at least one spinning pack is typically correlated with the viscosity of the molten polymer. When the viscosity of the molten polymer is unstable, an ejection amount of the molten polymer discharged from the spinneret becomes unstable. As a result, the thickness of the at least one yarn also becomes unstable (i.e., the quality of the at least one yarn also becomes unstable). To put it differently, when it is determined that the pressure of the molten polymer is stable, it is determined that the quality of the at least one yarn is stable. Therefore, usage of the pressure detection unit as the information detection unit is effective.

The viscosity of the molten polymer is correlated with the temperature of the molten polymer. When the temperature of the molten polymer is unstable, the viscosity of the molten polymer becomes unstable, with the result that the quality of the at least one yarn also becomes unstable as described above. When it is determined that the surface temperature of the spinneret is stable, it is determined that (i) the temperature of molten polymer discharged from the spinneret is stable and (ii) the quality of the at least one yarn is stable. Therefore, usage of a temperature detection unit, when provided as part of the information detection unit, is effective.

According to the first aspect of the invention, the yarn spinning system is arranged such that the information detection unit includes the pressure detection unit.

Typically, when the temperature detection unit is provided in the vicinity of the spinneret, the position adjustment of the temperature detection unit, etc. by an operator may be laborious because maintenance such as cleaning is frequently performed for the spinneret. Meanwhile, because the at least one spinning pack is typically replaced only when, e.g., the brand of the at least one yarn is changed, an operation is relatively less-frequently performed for the at least one spinning pack. Therefore, by providing the pressure detection unit in the vicinity of, e.g., the at least one spinning pack, the burden on the operator can be suppressed as compared to a case where the temperature detection unit is provided in the vicinity of the spinneret. According to this aspect, the temperature detection unit can be omitted because the pressure detection unit is provided. It is therefore possible to suppress the increase of the burden on the operator due to provision of the information detection unit.

According to the first aspect of the invention, the yarn spinning system is arranged such that the spinning apparatus further includes a pushing unit configured to push out the molten polymer toward the at least one spinning pack, and the pressure detection unit is provided between the pushing unit and the at least one spinning pack in a direction in which the molten polymer flows.

According to this aspect, being different from a case where, e.g., the pressure detection unit is provided in the at least one spinning pack, replacement of the pressure detection unit is not required even when the at least one spinning pack is replaced.

According to a second aspect of the invention, the yarn spinning system of the first aspect is arranged such that, when at least one predetermined standard condition is kept satisfied by the at least one determination parameter for a predetermined standard time, the first control unit determines that the quality of the at least one yarn is stable.

When it is determined that the quality of the at least one yarn is stable as soon as the at least one determination parameter satisfies the at least one standard condition, this determination may be incorrect. According to this aspect, when some time elapses after the at least one determination parameter satisfies the at least one standard condition, it is determined that the quality of the at least one yarn is stable. It is therefore possible to decrease the risk of incorrect determination.

According to a third aspect of the invention, the yarn spinning system of the second aspect is arranged such that the at least one determination parameter includes a detection result parameter regarding a value indicating the detection result, and the at least one standard condition includes a standard detection condition regarding whether the detection result parameter is within a predetermined standard detection range. In this aspect, when the detection result parameter is within the standard detection range, the first control unit determines that the detection result parameter satisfies the standard detection condition.

When the pressure in the at least one spinning pack is detected by the information detection unit, a value of this pressure is the detection result parameter. When a temperature detection unit is optionally provided and detects the surface temperature of the spinneret, a value of this temperature is the detection result parameter. This aspect makes it possible to determine whether the quality of the at least one yarn is stable in a simplified manner.

According to a fourth aspect of the invention, the yarn spinning system of the second or third aspect is arranged such that the at least one determination parameter includes at least one dispersion parameter regarding dispersion of a value indicating the detection result, and the at least one standard condition includes a standard dispersion condition regarding whether the at least one dispersion parameter is within a predetermined standard dispersion range. In this aspect, when the at least one dispersion parameter is within the standard dispersion range, the first control unit determines that the at least one dispersion parameter satisfies the standard dispersion condition.

For example, dispersion parameters include the dispersion of the value indicating the detection result, a standard deviation, or a variation coefficient (a value acquired by dividing the standard deviation by an average value). This aspect makes it possible to accurately determine whether the quality of the at least one yarn is stable.

According to a fifth aspect of the invention, the yarn spinning system of the fourth aspect is arranged such that the information detection unit includes the pressure detection unit configured to detect the pressure inside of the at least one spinning pack, and the at least one dispersion parameter includes a variation coefficient of the pressure.

For example, a standard deviation of the pressure inside of the at least one spinning pack may be monitored as one dispersion parameter. However, typically, the pressure inside of the at least one spinning pack may vary depending on the brand of the at least one yarn (thickness of the at least one yarn). Therefore, a standard dispersion range of the standard deviation may greatly vary. In this regard, because the variation coefficient is a value indicating the ratio of dispersion, a common standard value which does not depend on the brand of the at least one yarn may be provided as a standard dispersion range of the variation coefficient.

According to a sixth aspect of the invention, a yarn spinning system comprises the technical features of claim 6.

According to a seventh aspect of the invention, the yarn spinning system of the sixth aspect is arranged such that the spinning apparatus further includes a spinning beam in which a pack housing in which the at least one spinning pack is detachably housed is formed. In this aspect, as the spinning pack, the pack housing is able to house one spinning pack of one type among spinning packs of plural types for spinning out yarns that are different from one another in thickness, the first control unit includes a storage unit which is able to store information, and the storage unit stores information of the standard dispersion range regarding the variation coefficient as information shared between the spinning packs of the plural types.

This aspect makes it possible to keep the standard dispersion range constant irrespective of the thickness of each yarn. It is therefore possible to effectively simplify the management of the standard dispersion range.

According to an eighth aspect of the invention, a yarn spinning system comprises the technical features of claim 8.

According to the eighth aspect of the invention, the yarn spinning system is arranged such that the spinning apparatus includes the spinning beam in which a pack housing in which the at least one spinning pack is detachably housed is formed. In this aspect, the pack housing is able to house one spinning pack of one type among spinning packs of plural types for spinning out yarns that are different from one another in thickness, the first control unit includes a storage unit which is able to store information, and the storage unit stores information of the standard time as information shared between the spinning packs of the plural types.

This aspect makes it possible to effectively simplify the management of the standard time.

According to a ninth aspect of the invention, the yarn spinning system of the eighth aspect is arranged such that, when the at least one determination parameter does not satisfy the at least one standard condition after it is determined that the quality of the at least one yarn is stable, the first control unit determines that the quality of the at least one yarn is unstable.

According to this aspect, some measures can be taken when the quality of the at least one yarn is unstable again after the quality of the at least one yarn is stable.

According to a tenth aspect of the invention, the yarn spinning system of the ninth aspect is arranged such that the spinning apparatus includes spinning packs, the spinning packs include a first spinning pack configured to spin out a first yarn as the at least one yarn and a second spinning pack configured to spin out a second yarn which is different from the first yarn, the yarn spinning system further includes a cooling apparatus configured to cool the first yarn and the second yarn with use of cooling wind, the cooling apparatus includes: a first cooling cylinder which is provided to surround the first yarn and is configured to allow the cooling wind cooling the first yarn to pass through the first cooling cylinder; a second cooling cylinder which is provided to surround the second yarn and is configured to allow the cooling wind cooling the second yarn to pass through the second cooling cylinder; and a common duct which is provided upstream of the first cooling cylinder and the second cooling cylinder in a flow direction in which the cooling wind flows, the first cooling cylinder is provided to be close to a downstream end of the common duct in the flow direction as compared to the second cooling cylinder, and the information detection unit is provided to correspond to the first spinning pack.

When the first cooling cylinder is provided nearby the common duct, the cooling wind may be relatively easily supplied to the first cooling cylinder. Therefore, a spinneret of the first spinning pack may be cooled relatively more easily than a spinneret of the second spinning pack. In this case, the quality of the first yarn may be unlikely to become stable as compared to that of the second yarn. According to this aspect, information regarding the quality of the first yarn is detectable by the information detection unit. Because of this, when it is determined that the quality of the first yarn is stable, it is ensured that the quality of other yarns is also stable.

According to an eleventh aspect of the invention, the yarn spinning system of any one of the first to ninth aspects is arranged such that the spinning apparatus includes spinning packs, the spinning packs include a first spinning pack configured to spin out a first yarn as the at least one yarn and a second spinning pack configured to spin out a second yarn which is different from the first yarn, the information detection unit includes a first detection unit provided to correspond to the first spinning pack and a second detection unit provided to correspond to the second spinning pack, and as the quality determination, the first control unit is programmed to perform first quality determination with use of a detection result of the first detection unit and to perform second quality determination with use of a detection result of the second detection unit. In this aspect, the first quality determination is determination of whether the quality of the first yarn is stable, and the second quality determination is determination of whether the quality of the second yarn is stable.

When the spinning packs are provided, the credibility of the quality determination is further increased by performing the quality determination for two or more yarns.

According to a twelfth aspect of the invention, a spun yarn winding system includes: the yarn spinning system according to the first aspect; a spun yarn take-up winder configured to form a package by winding the at least one yarn; and a second control unit. In this aspect, the second control unit is programmed to perform, based on a result of the quality determination by the first control unit, at least one of control of the spun yarn take-up winder and determination of a grade of the package.

According to this aspect, the result of the quality determination can be reflected to the way of handling of the package.

According to the twelfth aspect of the invention, the spun yarn winding system is arranged such that the spun yarn take-up winder includes a bobbin switching mechanism arranged to be able to perform a bobbin switching operation in which at least one bobbin onto which the at least one yarn is wound is switched from a predetermined first bobbin to a second bobbin which is different from the first bobbin. In this aspect, when the first control unit determines that the quality of the at least one yarn is stable, the second control unit causes the bobbin switching mechanism to perform the bobbin switching operation.

The package formed before the quality of the at least one yarn becomes stable is required to be handled as a low-grade package. According to this aspect, the bobbin switching operation is performed when the quality of the at least one yarn becomes stable. Because of this, a package formed by winding the at least one yarn onto the first bobbin is handled as a low-grade package, and a package formed by winding the at least one yarn onto the second bobbin is handled as a high-grade package. As such, the state of the spun yarn take-up winder is automatically changeable between a state in which the low-grade package is formed to a state in which the high-grade package is formed.

According to a thirteenth aspect of the invention, the spun yarn winding system of the twelfth aspect is arranged such that, when the first control unit determines that the quality of the at least one yarn is unstable again after determining that the quality of the at least one yarn is stable, the second control unit causes the bobbin switching mechanism to perform the bobbin switching operation again.

According to this aspect, when the quality of the at least one yarn becomes unstable for some reason, the at least one bobbin onto which the at least one yarn is wound is replaced again. It is therefore possible to avoid a case where a low-quality yarn is wound onto the high-grade package.

According to a fourteenth aspect of the invention, the spun yarn winding system of the twelfth or thirteenth aspects further includes a notification unit configured to notify information. In this aspect, the second control unit is programmed to drive the notification unit based on the result of the quality determination by the first control unit.

According to this aspect, the operator, etc. is notified of information regarding the grade of the package which is being formed. It is therefore possible to prompt the operator to perform an operation regarding the management of the grade of the package.

### BRIEF DESCRIPTION OF THE DRAWINGS

FIG. 1 is a profile which schematically shows a spun yarn winding system of the embodiment.
FIG. 2 is a schematic representation of a yarn spinning system.
FIG. 3 is a side view of a cooling apparatus.
FIG. 4 is a cross section taken along a line IV-IV in FIG. 3.
FIG. 5 is a front view of a spun yarn take-up winder.
FIG. 6 illustrates a yarn shifting unit.
Each of FIGs. 7(a) and 7(b) illustrates a bobbin switching operation.
FIG. 8(a) is a graph showing changes in pressure at the inside of one spinning pack over time, and FIG. 8(b) is a graph showing changes of a variation coefficient of the pressure over time.
FIG. 9 is a table prepared for explaining results of quality determination.
FIG. 10 illustrates a spinning apparatus of a modification.

### DESCRIPTION OF THE PREFERRED EMBODIMENTS

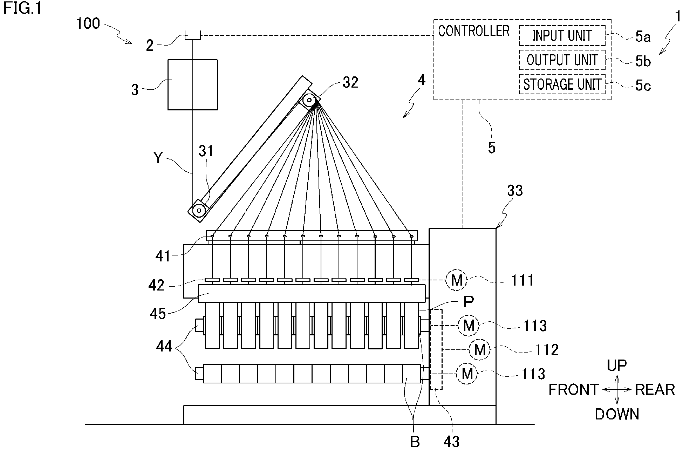

The following will describe an embodiment of the present invention. Hereinafter, directions shown in FIG. 1 will be consistently used as an up-down direction and a front-rear direction, for convenience of explanation. The up-down direction is a vertical direction in which the gravity acts. The front-rear direction is a direction in which each later-described bobbin holder 44 extends. A direction orthogonal to both the up-down direction and the front-rear direction will be consistently used as a left-right direction. Moreover, a direction in which a yarn Y runs will be consistently used as a yarn running direction.

### (Spun Yarn Winding System)

The following will outline a spun yarn winding system 1 of the present embodiment, with reference to FIG. 1. FIG. 1 is a profile which schematically shows the spun yarn winding system 1 of the present embodiment. The spun yarn winding system 1 is configured to form packages P by winding spun-out yarns Y onto bobbins B.

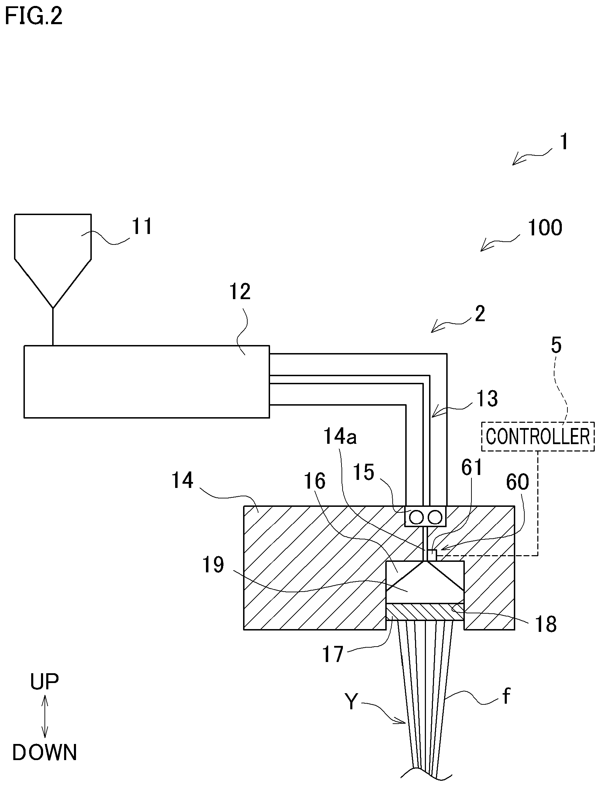

The spun yarn winding system 1 includes a spinning apparatus 2, a cooling apparatus 3, a spun yarn take-up winder 4, and a controller 5 (first control unit and second control unit in the present invention). The spinning apparatus 2 is configured to spin out the yarns Y made of synthetic fibers. The spinning apparatus 2 is configured to discharge molten polymer, which is a material of each yarn Y, as one or more filaments f (see FIG. 2). The cooling apparatus 3 is configured to form the yarns Y by cooling and solidifying the one or more filaments f by means of cooling wind. The spun yarn take-up winder 4 is configured to simultaneously form the packages P by winding the yarns Y onto the respective bobbins B. The controller 5 is configured to control the components of the spinning apparatus 2, cooling apparatus 3, and spun yarn take-up winder 4. In the present embodiment, a combination of the spinning apparatus 2, the cooling apparatus 3, and the controller 5 is equivalent to a yarn spinning system 100.

FIG. 1 illustrates one spinning apparatus 2, one cooling apparatus 3, one spun yarn take-up winder 4, and one controller 5. However, the disclosure is not limited to this. The spun yarn winding system 1 may include plural spinning apparatuses 2, plural cooling apparatuses 3, plural spun yarn take-up winders 4, and plural controllers 5. When the controllers 5 are provided, an integrated controller (not illustrated) configured to integrally control the controllers 5 may be provided.

### (Spinning Apparatus)

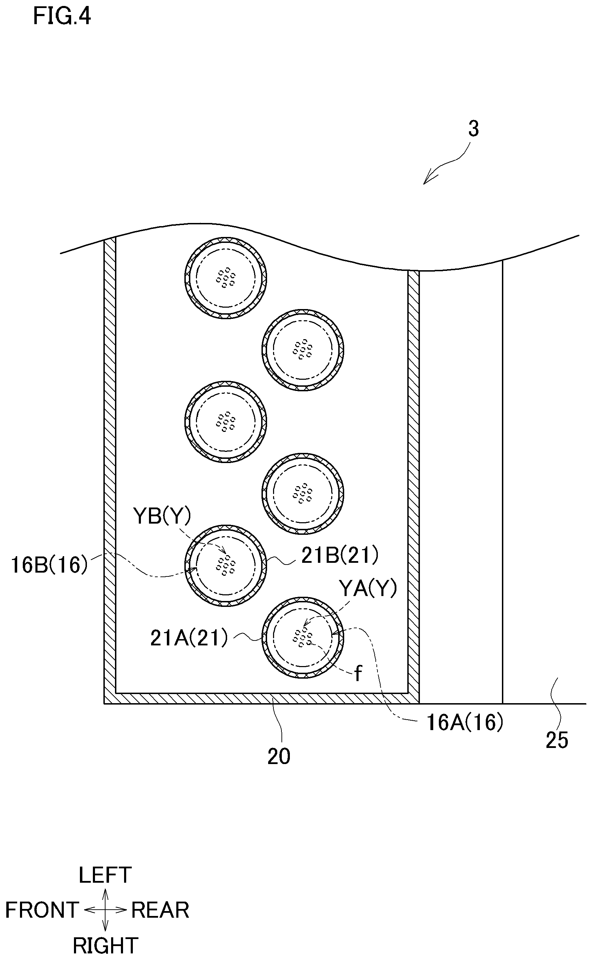

The following will describe the spinning apparatus 2, mainly with reference to a schematic representation of FIG. 2. For example, the spinning apparatus 2 includes a hopper 11, a melt pushing device 12, a polymer line 13, a spinning beam 14, gear pumps 15 (pushing units of the present invention: FIG. 2 illustrates only one gear pump 15), and spinning packs 16 (see two-dot chain lines in FIG. 4). Roughly speaking, a solid yarn material in the hopper 11 is melted by the melt pushing device 12 to be molten polymer, and the molten polymer is pushed out to the polymer line 13. Examples of the type of a yarn material include polyester, nylon 6, and nylon 66. The molten polymer which is pushed out to the polymer line 13 is then pushed out to the spinning packs 16 by the gear pumps 15 which are housed in the spinning beam 14. The molten polymer which is supplied to each spinning pack 16 corresponding to one gear pump 15 is discharged from a spinneret 17 provided at each spinning pack 16.

The hopper 11 is a funnel-shaped device into which polymer is poured. The melt pushing device 12, the polymer line 13, and the spinning beam 14 are respectively heated by unillustrated heaters so that the temperatures of the melt pushing device 12, the polymer line 13, and the spinning beam 14 are increased to a predetermined temperature. The melt pushing device 12 is configured to melt polymer, and to push out the polymer (molten polymer) to the polymer line 13. The polymer line 13 has branched passages (not illustrated), and is configured to supply molten polymer to the spinning packs 16. The spinning beam 14 has pack housings 18 each of which is concave and open downward. The gear pumps 15 are provided above the respective pack housings 18. In the up-down direction, one passage 14a in which molten polymer flows is formed between each gear pump 15 and each pack housing 18. The molten polymer is pushed out by the gear pump 15. The spinning packs 16 are detachably attached to the respective pack housings 18. Each pack housing 18 is able to house one spinning pack 16. When viewed in the up-down direction, the spinning packs 16 are provided in, e.g., a staggered manner (see two-dot chain lines in FIG. 4). In each spinning pack 16, an inflow space 19 into which molten polymer flows is provided. Below the inflow space 19, a filter (not illustrated) by which impurities in the molten polymer are removed is provided. Below the filter (At a lower end portion of each spinning pack 16), the spinneret 17 configured to discharge the molten polymer is provided. The spinneret 17 has nozzles (not illustrated) which penetrate the spinneret 17 in the up-down direction. Molten polymer which is supplied to the inflow space 19 by the gear pump 15 is pushed out downward through the nozzles of the spinneret 17, and then is discharged as one or more filaments f. The number of filaments f spun out from the spinneret 17 and the thickness of each filament f (i.e., the brand of the yarn Y) vary mainly depending on the number of the nozzles formed at the spinneret 17 and an aperture area of each nozzle. That is, as one spinning pack 16 of one type housed in each pack housing 18 is replaced with another spinning pack 16 of another type, the thickness of a spun-out yarn Y is changed. In other words, each pack housing 18 is able to house a spinning pack 16 of one type among spinning packs 16 of plural types for spinning out yarns Y that are different from one another in thickness.

### (Cooling Apparatus)

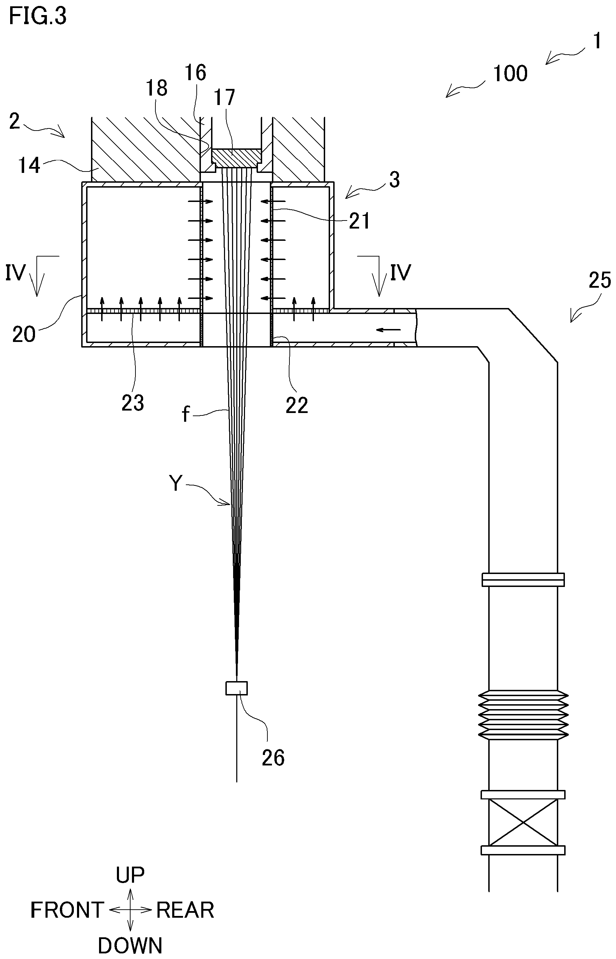

The following will describe the cooling apparatus 3 with reference to FIG. 3 and FIG. 4. FIG. 3 is a side view of the cooling apparatus 3. FIG. 4 is a cross section taken along a line IV-IV in FIG. 3. The cooling apparatus 3 is configured to cool and solidify molten polymer spun out from the spinning packs 16. The cooling apparatus 3 is provided below the spinning beam 14.

As shown in FIG. 3, the cooling apparatus 3 includes a box 20, cooling cylinders 21, and partitioning cylinders 22. An internal space of the box 20 is partitioned into an upper space and a lower space by a flow adjustment plate 23. The flow adjustment plate 23 is made of a material having flow adjustment capability, such as punching metal. The flow adjustment plate 23 is provided substantially horizontally. The cooling cylinders 21 are housed in the box 20. The cooling cylinders 21 are provided above the flow adjustment plate 23 (i.e., provided in the upper space). The cooling cylinders 21 are provided below the spinning packs 16. As shown in FIG. 4, the cooling cylinders 21 are provided in a staggered manner in the left-right direction to correspond to the arrangement of the spinning packs 16. A wall of each cooling cylinder 21 is, in the same manner as the flow adjustment plate 23, made of a material having flow adjustment capability such as punching metal. The partitioning cylinders 22 are provided below the flow adjustment plate 23 (i.e., provided in the lower space) in the box 20. The partitioning cylinders 22 are provided below the cooling cylinders 21. A wall of each partitioning cylinder 22 is, being different from the cooling cylinders 21, made of a material which does not allow air to pass therethrough. A yarn Y passes through the internal space of each cooling cylinder 21 provided immediately below a corresponding spinning pack 16 and the internal space of each partitioning cylinder 22 in order.

To a rear part of a lower portion of the box 20, a duct 25 (common duct of the present invention) is connected. The duct 25 is connected to an unillustrated compressed air source. The compressed air source feeds air for cooling the yarns Y to the inside of the duct 25. The air is supplied into the lower space of the box 20 through the duct 25. In a flow direction in which cooling wind flows, the duct 25 is provided upstream of the cooling cylinders 21 (i.e., upstream of later-described cooling cylinders 21A and 21B). As shown in FIG. 4, among the cooling cylinders 21, a cooling cylinder 21 (e.g., the cooling cylinder 21A) provided on the rear side in the front-rear direction are provided to be closer to a downstream end of the duct 25 in the flow direction than a cooling cylinder 21 (e.g., the cooling cylinder 21B) provided on the front side in the front-rear direction. In the present embodiment, for example, the downstream end of the duct 25 indicates a front end of the duct 25 in the front-rear direction. The cooling cylinder 21A is equivalent to a first cooling cylinder of the present invention. The cooling cylinder 21B is equivalent to a second cooling cylinder of the present invention. Among the above-described spinning packs 16, a spinning pack 16A provided immediately above the cooling cylinder 21A is equivalent to a first spinning pack of the present invention. Among the spinning packs 16, a spinning pack 16B provided immediately above the cooling cylinder 21B is equivalent to a second spinning pack of the present invention. Among the yarns Y, a yarn YA spun out from the spinning pack 16A is equivalent to a first yarn of the present invention. Among the yarns Y, a yarn YB spun out from the spinning pack 16B is equivalent to a second yarn of the present invention.

The cooling air which flows into the lower space of the box 20 passes through the flow adjustment plate 23 to be adjusted to flow upward, and flows into the upper space of the box 20. The air which flows into the upper space of the box 20 is adjusted while passing through the wall of each cooling cylinder 21. Subsequently, the air flows into each cooling cylinder 21. The air is blown to each yarn Y from the entire outer circumference of a corresponding cooling cylinder 21, so that each yarn Y is cooled in the corresponding cooling cylinder 21. In this regard, because the wall of each partitioning cylinder 22 does not allow the air to pass therethrough, the air does not flow directly into each partitioning cylinder 22 from the lower space of the box 20.

Below the cooling cylinder 21 and the partitioning cylinder 22, an oil guide 26 is provided (see FIG. 3). The oil guide 26 is configured to apply oil to a yarn Y in order to allow the yarn Y to smoothly run.

### (Spun Yarn Take-Up Winder)

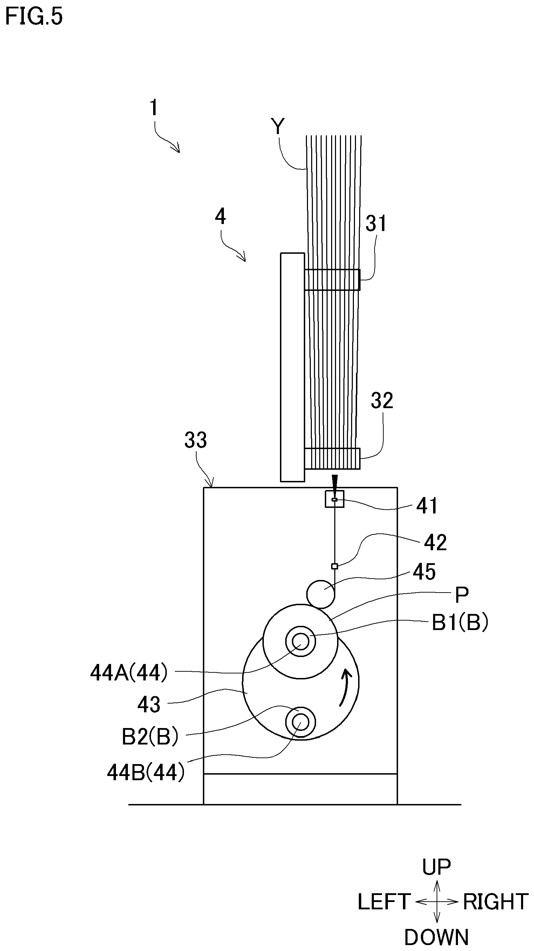

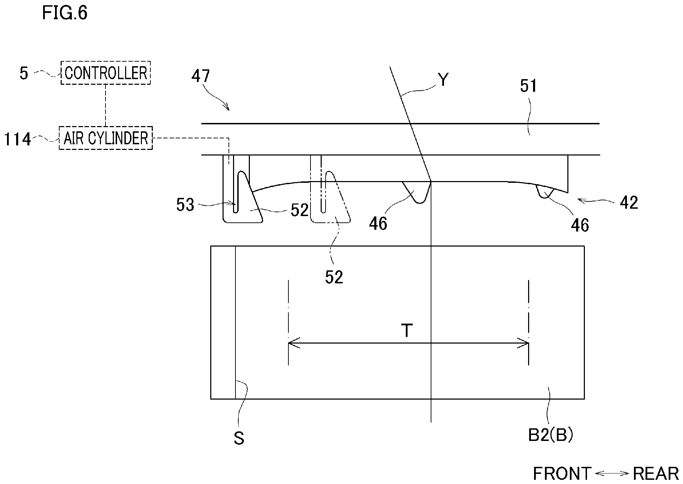

The following will describe the spun yarn take-up winder 4 with reference to FIG. 1, FIG. 5, and FIG. 6. FIG. 5 is a front view of the spun yarn take-up winder 4. FIG. 6 illustrates a later-described yarn shifting unit 47 (bobbin switching mechanism of the present invention).

As shown in FIG. 1 and FIG. 5, the spun yarn take-up winder 4 includes a first godet roller 31, a second godet roller 32, and a winding device 33. The first godet roller 31 is provided for taking up yarns Y. An axial direction of the first godet roller 31 is substantially parallel to the left-right direction. The first godet roller 31 is provided downstream of the oil guide 26 in the yarn running direction. The first godet roller 31 is provided above a front end portion of the winding device 33. The first godet roller 31 is rotationally driven by an unillustrated motor. The second godet roller 32 is provided for causing the yarns Y taken up by the first godet roller 31 to run above the winding device 33. An axial direction of the second godet roller 32 is substantially parallel to the left-right direction. The second godet roller 32 is provided above and behind the first godet roller 31. The second godet roller 32 is rotationally driven by an unillustrated motor.

The winding device 33 is configured to form the packages P by winding the yarns Y onto the respective bobbins B. The winding device 33 is provided below the first godet roller 31 and the second godet roller 32. As shown in FIG. 1 and FIG. 5, the winding device 33 includes fulcrum guides 41, traverse guides 42, a turret 43, two bobbin holder 44, and a contact roller 45.

Each of the fulcrum guides 41 is a guide about which a yarn Y is traversed by each traverse guide 42. The fulcrum guides 41 are aligned in the front-rear direction. The fulcrum guides 41 are provided for the respective yarns Y.

The traverse guides 42 are provided to traverse the respective yarns Y. The traverse guides 42 are aligned in the front-rear direction. The traverse guides 42 are provided to correspond to the respective fulcrum guides 41. Each traverse guide 42 is, e.g., a known blade-type traverse guide. Each traverse guide 42 includes two blade guides 46 (see FIG. 6) rotating in an opposite direction to each other. The blade guides 46 are driven by, e.g., a traverse motor 111 (see FIG. 1). Because of this, each yarn Y is traversed about a corresponding fulcrum guide 41 in the front-rear direction by the blade guides 46. Each yarn Y is traversed in the front-rear direction within a predetermined range (see a traverse area T in FIG. 6). The later-described yarn shifting unit 47 (see FIG. 6) is provided in the vicinity of the traverse guides 42.

The turret 43 is a disc-shaped member. An axial direction of the turret 43 is substantially parallel to the front-rear direction. The turret 43 is rotationally driven by a turret motor 112 (see FIG. 1). Each of the two bobbin holders 44 supports the bobbins B so that the bobbins B are aligned in the front-rear direction. An axial direction of each bobbin holder 44 is substantially parallel to the front-rear direction. The two bobbin holders 44 are rotatably supported by the turret 43. When viewed in the front-rear direction, the two bobbin holders 44 are provided to oppose each other over a center point of the turret 43. To be more specific, for example, when one bobbin holder 44 is positioned at the highest part of the turret 43, the other bobbin holder 44 is positioned at the lowest part of the turret 43. Each bobbin holder 44 rotatably supports the bobbins B which are provided for the respective yarns Y. To each bobbin holder 44, the bobbins B provided for the respective yarns Y are attached to be lined up in the front-rear direction. Each of the two bobbin holders 44 is rotationally driven by an individual winding motor 113 (see FIG. 1). The contact roller 45 is a roller having an axis substantially in parallel to the front-rear direction. The contact roller 45 is provided immediately above the upper bobbin holder 44. The contact roller 45 is configured to make contact with the surfaces of the packages P supported by the upper bobbin holder 44. With this, the contact roller 25 applies a contact pressure to the surfaces of the unfinished packages P, to adjust the shape of each package P.

When the upper bobbin holder 44 is rotationally driven, the yarns Y traversed by the traverse guides 42 are wound onto the bobbins B, with the result that the packages P are formed (winding process). When the formation of the packages P is completed, the turret 43 rotates to switch over the upper and lower positions of the two bobbin holders 44. As a result, the bobbin holder 44 having been at the lower position is moved to the upper position. This allows the yarns Y to be wound onto the bobbins B attached to the bobbin holder 44 having been moved to the upper position, to form packages P. Meanwhile, the bobbin holder 44 to which fully-formed packages P are attached is moved to the lower position and the packages P are collected by, e.g., an unillustrated package collector.

As shown in FIG. 6, the yarn shifting unit 47 is provided in the vicinity of the traverse guides 42. The yarn shifting unit 47 is able to change bobbins B onto which the yarns Y are wound. For example, in the winding device 33 shown in FIG. 5, assume that (i) the yarns Y are respectively wound onto bobbins B1 which are attached to one bobbin holder 44A and (ii) bobbins B2 are attached to the other bobbin holder 44B. In this case, by performing a predetermined operation together with the turret 43 and the bobbin holders 44, the yarn shifting unit 47 is able to switch the state of the winding device 33 between a state in which the yarns Y are wound onto the bobbins B1 and a state in which the yarns Y are wound onto the bobbins B2 (this operation will be detailed later). For the sake of convenience, an operation of the winding device 33 to change the bobbins B onto which the yarns Y are wound is referred to as a bobbin switching operation.

As shown in FIG. 6, the yarn shifting unit 47 includes a guide member 51 extending in the front-rear direction and holding units 52 which are provided to correspond to the respective yarns Y. Each of the holding units 52 includes a retaining groove 53 provided for retaining a yarn Y. The holding units 52 are simultaneously moved in the front-rear direction by, e.g., an air cylinder 114 (see FIG. 6). With this arrangement, each holding unit 52 is movable between a retracted position (indicated by a solid line in FIG. 6) provided outside a corresponding traversing area T and a capturing position (indicated by a two-dot chain line in FIG. 6) provided inside the traversing area T. To be more specific, for example, the air cylinder 114 is driven as an electromagnetic valve (not illustrated) provided for supplying and exhausting compressed air to the air cylinder 114 is opened or closed.

### (Controller)

The controller 5 is, e.g., a typical computer device. As shown in FIG. 1, the controller 5 includes an input unit 5a provided for inputting information, an output unit 5b provided for outputting information, and a storage unit 5c provided for storing information. The input unit 5a includes, e.g., at least one of a button (not illustrated), a touch panel (not illustrated), and a keyboard (not illustrated). The output unit 5b includes, e.g., an alarm lamp (not illustrated) and/or a display (not illustrated). The output unit 5b may further include a speaker (not illustrated) capable of outputting sound. The storage unit 5c stores sets of information used for activating the spun yarn winding system 1.

The controller 5 is able to control the components of the spun yarn winding system 1. For example, the controller 5 is configured to control operations of the above-described traverse motor 111, turret motor 112, winding motor 113 (see FIG. 1), and air cylinder 114 (see FIG. 6).

### (Bobbin Switching Operation)

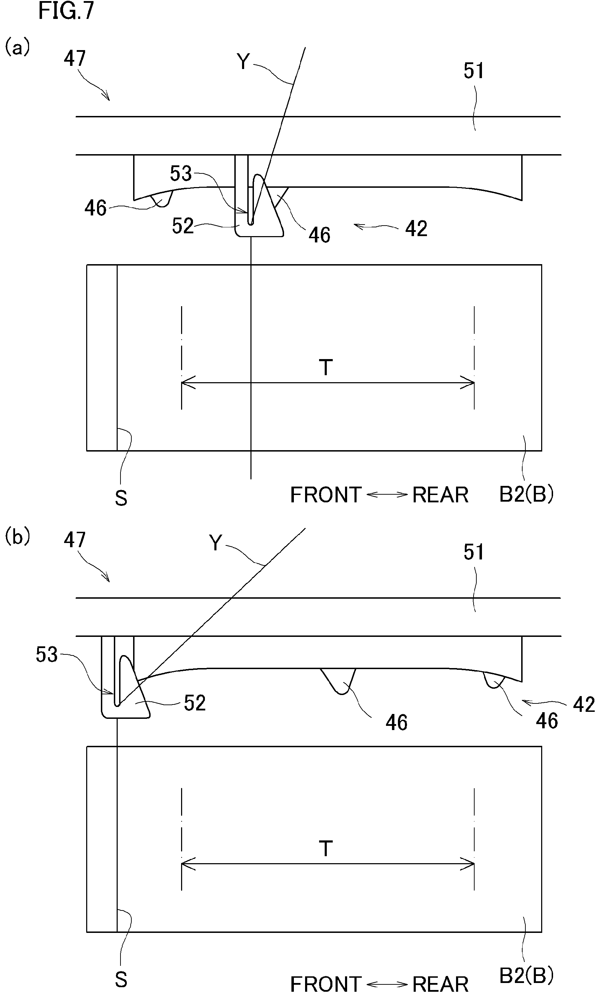

The following will describe the bobbin switching operation mainly with reference to FIG. 6 and FIGs. 7(a) and 7(b). Each of FIG. 6 and FIGs. 7(a) and 7(b) illustrates only one holding unit 52. However, actually, all holding units 52 operate simultaneously. For example, when formation of packages P (see FIG. 5) is completed, the controller 5 controls the operations of the turret motor 112, the winding motor 113, and the air cylinder 114 to cause the turret 43, the bobbin holders 44, and the yarn shifting unit 47 to perform the bobbin switching operation described below. In the present embodiment, a combination of the turret 43, the bobbin holders 44, and the yarn shifting unit 47 is equivalent to the bobbin switching mechanism of the present invention.

To begin with, when the formation of the packages P shown in FIG. 5 (i.e., winding of yarns Y onto the bobbins B1) is completed, the controller 5 controls the turret motor 112 to rotate the turret 43. As the turret 43 is rotated, each bobbin B2 is moved to the vicinity of a corresponding traverse guide 42 as shown in FIG. 6. In this state, the holding unit 52 is at the retracted position. Furthermore, each yarn Y is still connected to a corresponding package P and is traversed by the traverse guide 42. Subsequently, the controller 5 controls the winding motor 113 to rotate the bobbin holder 44B onto which the bobbin B2 is attached. The controller 5 then controls the operation of the air cylinder 114 to move the holding unit 52 from the retracted position to the capturing position (see FIG. 7(a)). Because of this, the traversed yarn Y enters a retaining groove 53 of the holding unit 52 so as to be captured by the holding unit 52. As the yarn Y is retained by the holding unit 52, the yarn Y is detached from the traverse guide 42. Subsequently, the controller 5 controls the operation of the air cylinder 114 to move the holding unit 52 from the capturing position to the retracted position (see FIG. 7(b)). Because of this, the yarn Y is hooked by a slit S formed at an end portion of the bobbin B2 in the axial direction. As the tension of the yarn Y hooked by the slit S increases, the yarn Y is broken. Because of this, the winding of the yarns Y onto the bobbins B1 ends, and winding of the yarns Y onto the bobbin B2 can be started. Subsequently, the controller 5 controls the operation of the air cylinder 114 to move the holding unit 52 from the retracted position to the capturing position (not illustrated). During this step, each yarn Y is captured by the traverse guide 42 and starts to be traversed. Because of this, the winding of the yarns Y onto the bobbins B2 starts. The bobbin switching operation executed by the bobbin switching mechanism is executed in this way.

In this regard, the quality of each yarn Y (e.g., dispersion of thickness of each yarn Y) spun out from the spinning apparatus 2 may vary depending on various factors such as the temperature of each spinneret 17. Especially, immediately after the yarn Y starts to be spun out from the spinning apparatus 2, the quality of the yarn Y is unstable because the temperature of the spinneret 17, etc. is not stable. To be more specific, the temperature of molten polymer is correlated with the viscosity of the molten polymer. When the temperature of the molten polymer is unstable, the viscosity of the molten polymer is also unstable. Therefore, the thickness of each filament f spun out from the spinneret 17 becomes unstable. As a result, the quality of the yarn Y becomes unstable. It takes some time for the quality of the yarn Y to become stable after the yarn Y starts to be spun out from the spinning apparatus 2. A yarn Y spun out before its quality becomes stable is typically worse in quality than a yarn Y spun out after its quality becomes stable. Therefore, these yarns Y need to be handled differently from each other. For example, a yarn Y spun out before its quality becomes stable may be wound onto one above-described bobbin B1 and a yarn Y spun out after its quality becomes stable may be wound onto one above-described bobbin B2. In this case, a package P formed by winding the yarn Y onto the bobbin B1 is handled as a low-grade package P.

Traditionally, the quality of yarns Y which start to be spun out from the spinning apparatus 2 is visually determined by an operator, etc. or is managed based on whether a predetermined time elapses after the yarns Y start to be spun out. However, when the determination of whether the quality of the yarns Y is stable (hereinafter, this determination will be referred to as quality determination) is performed by the operator, the burden on the operator is disadvantageously high. Meanwhile, when the management based on the elapse of time is performed, the following problems may occur. That is, a time required from the start of yarn spinning from the spinning apparatus 2 to the stabilization of the quality of the yarns Y may vary depending on the brands of the yarns Y. It is therefore necessary to manage or set the above-described predetermined time of each brand, with the result that the burden on the operator may be increased. Alternatively, when the above-described predetermined time elapses before the quality of the yarns Y actually becomes stable, it may be incorrectly determined that the quality of the yarns Y becomes stable. In the present embodiment, the spun yarn winding system 1 is structured as described below in order to determine, without needing human workforce, whether the quality of yarns Y is stable.

### (Details)

As shown in FIG. 2, the spinning apparatus 2 includes an information detection unit 60 which is able to detect information regarding the quality of yarns Y. For example, the information detection unit 60 includes a pressure sensor 61 (pressure detection unit of the present invention) configured to detect the pressure of later-described molten polymer which is pushed out by the gear pumps 15 and flows into the spinning packs 16. Among the spinning packs 16, the pressure sensor 61 is provided in the vicinity of, e.g., the spinning pack 16A (see FIG. 4) provided above the cooling cylinder 21A. The pressure sensor 61 is provided between one gear pump 15 and the spinning pack 16A (i.e., provided downstream of the gear pump 15 and upstream of the spinning pack 16A) in, e.g., a direction in which molten polymer flows. For example, the pressure sensor 61 may be provided in or nearby the passage 14a. The pressure sensor 61 is configured to transmit a signal indicating a detection result to the controller 5. The pressure sensor 61 is, e.g., a known sensor including an unillustrated diaphragm and a pressure-sensitive element. However, the structure of the pressure sensor 61 is not limited to this.

The pressure of the above-described molten polymer (hereinafter, this pressure will be simply referred to as the pressure) is typically correlated with the viscosity of the molten polymer. When the viscosity of the molten polymer is unstable, an ejection amount of the molten polymer discharged from the spinnerets 17 becomes unstable. As a result, the thickness of the yarns Y also becomes unstable (i.e., the quality of the yarns Y also becomes unstable). To put it differently, it can be determined that the quality of the yarns Y is stable when the pressure is stable.

The storage unit 5c of the controller 5 stores, e.g., the brand of each yarn Y and target values (an upper limit value and a lower limit value) of the pressure in association with each other. The storage unit 5c further stores, e.g., an upper limit value of a variation coefficient (details thereof will be given later) of the pressure. These values are set in such a way that, e.g., the operator operates the input unit 5a.

### (Quality Determination)

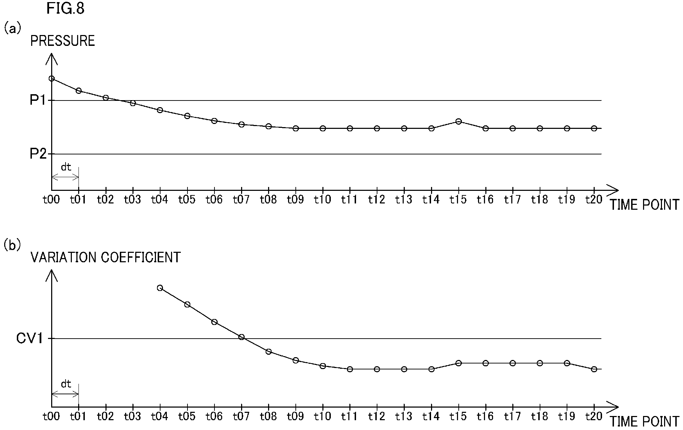

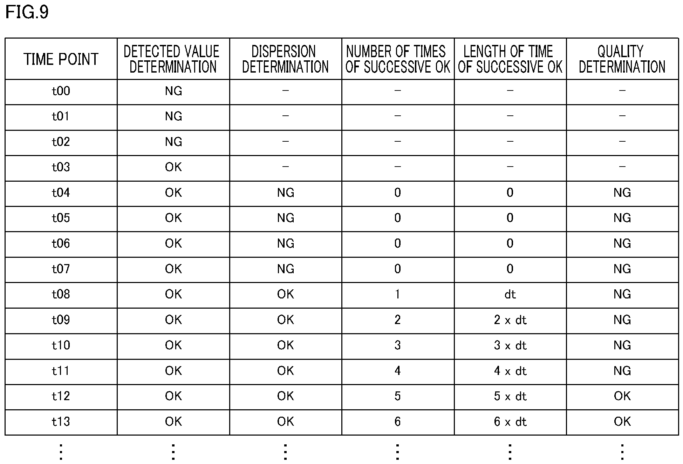

The following will describe the steps of the quality determination of quality of a yarn Y by the controller 5 (i.e., the determination of whether the quality of a yarn Y spun out from one spinning pack 16 provided in the vicinity of the pressure sensor 61 is stable), with reference to FIGs. 8(a) and 8(b) and FIG. 9. In the present embodiment, the phrase "the quality of a/the yarn Y (yarns Y) is stable" indicates that the variation of thickness of a/the yarn Y (yarns Y) over time is small. FIG. 8(a) is a graph showing changes in pressure at the inside of the spinning pack 16 over time. The horizontal axis of the graph indicates time points (to be more specific, time points t00 to t20). The vertical axis of the graph indicates the pressure in the spinning pack 16. FIG. 8(b) is a graph showing changes of the variation coefficient of the pressure in the spinning pack 16 over time. The horizontal axis of the graph indicates time points (to be more specific, time pints t00 to t20). The vertical axis of the graph indicates the variation coefficient. FIG. 9 is a table prepared for explaining results of the quality determination. FIG. 9 shows the following items (these items will be detailed later): time points; a result of detected value determination at each time point; each result of dispersion determination; the number of frequencies of successive OK; the length of time of successive OK; and each result of the quality determination. For the sake of convenience, FIG. 9 shows only determination results between the time point t00 and the time point t13. When the quality determination is performed, the controller 5 functions as a first control unit of the present invention.

As described above, the storage unit 5c stores an upper limit value (see P1 in FIG. 8(a)) and lower limit value (see P2 in FIG. 8(a)) of the pressure in correspondence with the brand of each yarn Y. Furthermore, the storage unit 5c stores an upper limit value (see CV1 in FIG. 8(b)) of the variation coefficient. The information of CV1 is stored in the storage unit 5c as information shared between spinning packs 16 of plural types (i.e., between various brands). The storage unit 5c may further store a lower limit value (e.g., zero) of the variation coefficient. The information regarding the lower limit value of the variation coefficient may not be stored in the storage unit 5c. In this case, the lower limit value of the variation coefficient is substantially zero because the variation coefficient is typically larger than zero.

The storage unit 5c further stores a predetermined standard time. Roughly speaking, the standard time indicates the length of time which is a standard used for determining that the quality of the yarn Y becomes stable after the quality of the yarn Y starts to become stable (details will be given later). The standard time is, e.g., stored in the storage unit 5c as information shared between the spinning packs 16 of plural types (i.e., between various brands).

In the present embodiment, an initial state is a state in which (i) the yarn Y starts to be spun out from the spinning apparatus 2 and (ii) the yarn Y starts to be wound onto one bobbin B1 by the spun yarn take-up winder 4. In this regard, although execution of predetermined yarn threading is required after the yarn Y starts to be spun out from the spinning apparatus 2 and before the yarn Y starts to be wound by the spun yarn take-up winder 4, the description of the yarn threading will be omitted.

For example, after the yarn threading is completed and the yarn Y starts to be wound onto the bobbin B1, the controller 5 acquires information of a detection result by the pressure sensor 61 each time a predetermined unit time dt (see FIGs. 8(a) and 8(b)) elapses. Based on the information of the detection result, the controller 5 then calculates and stores a detected value of the pressure (hereinafter, this will be referred to as a detected pressure value; this value is equivalent to one of determination parameters and a detection result parameter in the present invention) and the variation coefficient of the pressure (this coefficient is equivalent to one of determination parameters and a dispersion parameter in the present invention). The detected pressure value is a value calculated by using the detection result acquired by the pressure sensor 61. The variation coefficient of the pressure is acquired by dividing a standard deviation of the pressure by an average value of the pressure, and indicates the dispersion of the pressure. In this regard, the average value of the pressure indicates, e.g., an average value (i.e., moving average value) of detected pressure values which are acquired predetermined times in predetermined length of time. For example, the controller 5 acquires information of a detection result by the pressure sensor 61 five times during the time points t00 to t04. The controller 5 then calculates plural (five in this case) detected pressure values acquired during the time points t00 to t04, and calculates the average value and standard deviation of the pressure at the time point t04 based on the detected pressure values. Subsequently, the controller 5 calculates the variation coefficient of the pressure at the time point t04 based on this average value and this standard deviation. That is, in this example, the variation coefficient of the pressure is calculated for the first time (see FIG. 8(b)) when the detected pressure values regarding the time points t00 to t04 are acquired. Alternatively, the controller 5 may directly calculate the variation coefficient of the pressure by using the five detected pressure values acquired between the time point t00 and the time point t04. After that, the controller 5 similarly calculates the variation coefficient of the pressure at the time point t05 by using five detected pressure values acquired between the time point t01 and the time point t05. In other words, the controller 5 calculates the variation coefficient of the pressure at each time point by using a predetermined number of detected pressure values.

Each time the unit time dt elapses, the controller 5 determines whether each of the latest detected pressure value and the latest variation coefficient is within a predetermined range. To be more specific, the controller 5 performs the detected value determination (i.e., determination of whether a standard detection condition is satisfied) of whether the detected pressure value is within a range (standard detection range) between the upper limit value (see P1 in FIG. 8(a)) and the lower limit value (see P2 in FIG. 8(b)). In an example of FIG. 8(a), because each of detected pressure values between the time point t00 and the time point t02 is not within the standard detection range, the controller 5 determines (see FIG. 9) that each of the detected pressure values does not satisfy (i.e., the detected pressure values indicate "NG") the standard detection condition (standard condition of the present invention). Because each detected pressure value is within the standard detection range after the time point t03, the controller 5 determines (see FIG. 9) that the detected pressure value satisfies the standard detection condition (i.e., the detected pressure value is "OK"). The controller 5 further performs the dispersion determination (i.e., determination of whether the standard condition is satisfied) of whether the variation coefficient is within a range (standard dispersion range) between an upper limit value (see CV1 in FIG. 8(b)) and a lower limit value (e.g., zero). In an example of FIG. 8(b), the controller 5 does not perform the dispersion determination (see "-" in FIG. 9) between the time point t00 and the time point t03 because the variation coefficient is not acquired. Because the variation coefficient is not within the standard dispersion range between the time point t04 and the time point t07, the controller 5 determines that (i.e., determines that the variation coefficient is "NG"; see FIG. 9) the variation coefficient does not satisfy a standard dispersion condition (standard condition of the present invention). Because the variation coefficient is within the standard dispersion range after the time point t08, the controller 5 determines that (i.e., determines that the variation coefficient is "OK"; see FIG. 9) the variation coefficient satisfies the standard dispersion condition.

When both results of (i) the determination of the detected pressure value and (ii) the determination of the variation coefficient indicate "OK", the controller 5 increases the number of frequencies of successive determination of OK (i.e., the number of frequencies of successive OK in FIG. 9) by one. When at least one of the determination of the detected pressure value and the determination of the variation coefficient is "NG", the controller 5 resets the number of frequencies of successive OK to zero. In an example of FIG. 9, the controller 5 does not calculate the number of frequencies of successive OK between the time point t00 and the time point t03 because the determination of the variation coefficient is not performed. The controller 5 maintains the total number of frequencies of successive OK at zero because the variation coefficient is determined as "NG" between the time point t04 and the time point t07. The controller 5 updates the number of frequencies of successive OK because the determination of the detected pressure value and the determination of the variation coefficient are "OK" after the time point t08. The number of frequencies of successive OK may be considered as time (i.e., the length of time of successive OK) in which the determination of the detected pressure value and the determination of the variation coefficient are maintained at "OK". For example, the controller 5 may simply acquire, as the length of time of successive OK, time which is acquired by multiplying the unit time dt by the number of frequencies of successive OK (see FIG. 9).

The controller 5 further performs the quality determination based on whether the number of frequencies of successive OK is equal to or larger than predetermined standard frequencies (or whether the length of time of successive OK is equal to or longer than the standard time). The standard frequencies indicate, e.g., a value (an integer) acquired by dividing the standard time by the unit time dt. In this regard, the description below is given on the premise that the standard frequencies indicate five. In an example of FIG. 9, the controller 5 does not perform the quality determination between the time point t00 and the time point t03 because the number of frequencies of successive OK is not calculated. The controller 5 determines that each result of the quality determination indicates "NG" between the time point t04 and the time point t11 because the number of frequencies of successive OK is smaller than five. The controller 5 determines that each result of the quality determination indicates "OK" after the time point t12 because the number of frequencies of successive OK is equal to or larger than five. When the result of the quality determination indicates "OK", the quality of the yarn Y is stable. Roughly speaking, when the number of frequencies of successive OK is equal to or larger than the predetermined standard frequencies, the above-described length of time of successive OK is equal to or longer than the standard time. Alternatively, the controller 5 may actually calculate the length of time of successive OK based on the number of frequencies of successive OK. Furthermore, when the length of time of successive OK is equal to or longer than the standard time, it may be determined that a result of the quality determination indicates "OK". In other words, each of (i) the case where the number of frequencies of successive OK is equal to or larger than the standard frequencies and (ii) the case where the length of time of successive OK is equal to or longer than the standard time is equivalent to a case where a predetermined standard condition is kept satisfied by one determination parameter for a predetermined standard time.

When the result of the quality determination indicates "OK", the controller 5 finally controls and causes the spun yarn take-up winder 4 to execute the above-described bobbin switching operation. When controlling the spun yarn take-up winder 4, the controller 5 functions as the second control unit of the present invention. With this arrangement, a yarn Y before its quality becomes stable can be wound onto one bobbin B1 (first bobbin of the present invention), and a yarn Y after its quality becomes stable can be wound onto one bobbin B2 (second bobbin of the present invention).

As such, the controller 5 can perform the quality determination with use of a detection result by the pressure sensor 61. It is therefore possible to determine whether the quality of each yarn Y is stable, without needing human workforce. A result of the quality determination can be reflected to the way of handling of each package P. Each package P formed by winding the yarn Y onto the bobbin B1 can be handled as a low-grade package P, and each package P formed by winding the yarn Y onto the bobbin B2 can be handled as a high-grade package P. As such, the state of the spun yarn take-up winder 4 is automatically changeable between a state in which low-grade packages P are formed to a state in which high-grade packages P are formed.

The pressure of molten polymer is typically correlated with the viscosity of the molten polymer. When the viscosity of the molten polymer is unstable, an ejection amount of the molten polymer discharged from the spinnerets 17 becomes unstable. As a result, the thickness of the yarns Y also becomes unstable (i.e., the quality of the yarns Y also becomes unstable). To put it differently, when it is determined that the pressure of the molten polymer is stable, it is determined that the quality of the yarns Y is stable. Therefore, usage of the pressure sensor 61 as the information detection unit 60 is effective.

When a temperature sensor (later-described temperature sensor 62) is provided in the vicinity of the spinnerets 17, the position adjustment of the temperature sensor, etc. by the operator may be laborious because the maintenance such as cleaning is frequently performed for the spinnerets 17. Meanwhile, because each spinning pack 16 is typically replaced only when, e.g., the brand of each yarn Y is changed, an operation is relatively less-frequently performed for the spinning pack 16. Therefore, by providing the pressure sensor 61 in the vicinity of the spinning pack 16, the burden on the operator can be suppressed as compared to the case where the temperature sensor is provided in the vicinity of the spinnerets 17. In the present invention, the temperature sensor can be omitted because the pressure sensor 61 is provided. It is therefore possible to suppress the increase of the burden on the operator due to provision of the information detection unit 60.

In the present embodiment, being different from a case where the pressure sensor 61 is provided in the spinning pack 16, replacement of the pressure sensor 61 is not required even when the spinning pack 16 is replaced.

When some time elapses after one determination parameter satisfies the standard condition, it is determined that the quality of the yarn Y becomes stable. It is therefore possible to decrease the risk of incorrect determination.

The determination parameters include the detected value of the pressure, which is equivalent to the detection result parameter. When the detected value of the pressure is within the standard detection range, the controller 5 determines that the detected value of the pressure satisfies the standard detection condition. It is therefore possible to determine whether the quality of the yarn Y is stable in a simplified manner.

The determination parameters include the variation coefficient of the pressure, which is equivalent to the dispersion parameter. When the variation coefficient of the pressure is within the standard dispersion range, the controller 5 determines that the variation coefficient of the pressure satisfies the standard dispersion condition. It is therefore possible to accurately determine whether the quality of each yarn Y is stable. Because the variation coefficient is a value indicating the ratio of dispersion, a common standard value which does not vary between the brands of yarns Y may be provided as the standard dispersion range of the variation coefficient. This makes it possible to keep the standard dispersion range constant irrespective of the thickness of each yarn Y. It is therefore possible to effectively simplify the management of the standard dispersion range.

The standard time is stored in the storage unit 5c as information shared between the spinning packs 16 of plural types (i.e., between various brands). It is therefore possible to effectively simplify the management of the standard time.

The pressure sensor 61 is provided in the vicinity of the spinning pack 16A. The spinning pack 16A is provided immediately above the cooling cylinder 21A which is provided nearby the downstream end of the duct 25 in the flow direction of cooling wind. When the cooling cylinder 21A is provided nearby the duct 25, cooling wind may be relatively easily supplied to the cooling cylinder 21A. Therefore, a spinneret 17 of the spinning pack 16A (see FIG. 4) may be cooled relatively more easily than a spinneret 17 of the spinning pack 16B (see FIG. 4). In this case, the quality of the yarn YA (see FIG. 4) may be unlikely to become stable as compared to that of the yarn YB (see FIG. 4). In the present invention, the information regarding the quality of the yarn YA is detectable by the pressure sensor 61. Because of this, when it is determined that the quality of the yarn YA is stable, it is ensured that the quality of other yarns Y is also stable.

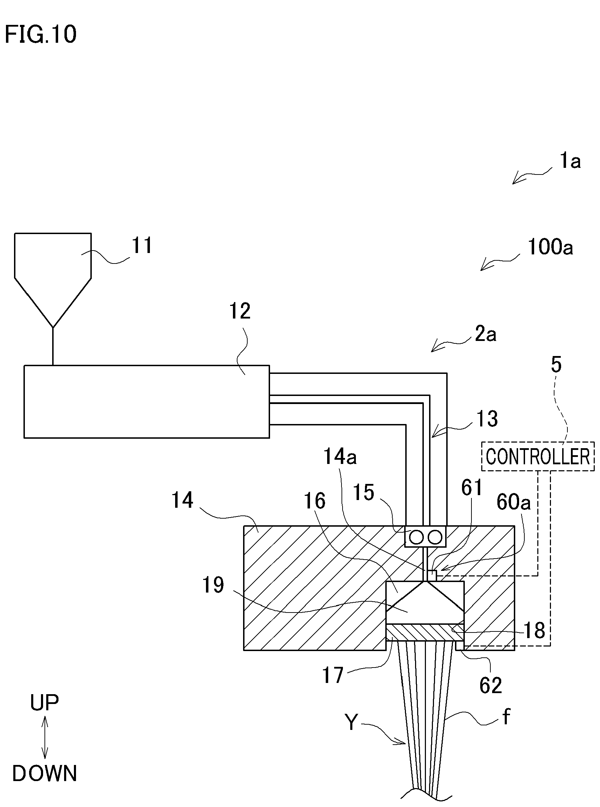

The following will describe modifications of the above-described embodiment. The members identical with those in the embodiment above will be denoted by the same reference numerals and the explanations thereof are not repeated.
(1) In the embodiment above, the controller 5 is configured to use the variation coefficient of the pressure as the dispersion parameter. However, the disclosure is not limited to this. The controller 5 may be configured to use, e.g., the standard deviation of the pressure as the dispersion parameter. In this regard, the standard deviation of the pressure may greatly vary depending on the brand of each yarn Y. Therefore, when the standard deviation of the pressure is used as the dispersion parameter, the controller 5 may be preferably arranged to be able to individually set the standard dispersion range of the standard deviation of the pressure for the brand of each yarn Y. The controller 5 may be arranged to be able to individually set the standard dispersion range for the brand of each yarn Y even when the variation coefficient of the pressure is used as the dispersion parameter in the same manner as in the embodiment above.
(2) In the embodiment above, the controller 5 is configured to use each of the detection result parameter and the dispersion parameter as the determination parameters. However, the disclosure is not limited to this. As the determination parameters, the controller 5 may be configured to use only one of the detection result parameter and the dispersion parameter. For example, the controller 5 may be configured to use only the variation coefficient of the pressure as the determination parameters. In this case, because only the standard dispersion range shared between the brands of yarns Y is used as the standard condition, the management of the standard condition is especially simplified.
(3) In the embodiment above, the information detection unit 60 includes only the pressure sensor 61. However, the disclosure is not limited to this. For example, in a spun yarn winding system 1a shown in FIG. 10, an information detection unit 60a included in a spinning apparatus 2a of a yarn spinning system 100a may include the temperature sensor 62 (temperature detection unit of the present invention) in addition to the pressure sensor 61. The temperature sensor 62 may be, e.g., a known thermocouple. The temperature sensor 62 may be provided to be in contact with, e.g., a lower surface or side surface of the spinneret 17 of the spinning pack 16A (see FIG. 4). The controller 5 may be configured to acquire at least one of the detection result parameter and the dispersion parameter based on a detection result by the temperature sensor 62, and to use the at least one of those for the quality determination. The viscosity of molten polymer is correlated with the temperature of the molten polymer. When the temperature of the molten polymer is unstable, the viscosity of the molten polymer becomes unstable, with the result that the quality of the yarns Y also becomes unstable as described above. When it is determined that a surface temperature of each spinneret 17 is stable, it is determined that (i) the temperature of molten polymer discharged from each spinneret 17 is stable and (ii) the quality of each yarn Y is stable. Therefore, usage of the temperature sensor 62 as the information detection unit 60a is effective. Alternatively, the information detection unit 60a may include only the temperature sensor 62. In other words, the information detection unit 60a may include at least one of the pressure sensor 61 and the temperature sensor 62.
(4) In the embodiment above, the information detection unit 60 (or the information detection unit 60a) is provided in the vicinity of the spinning pack 16A provided immediately above the cooling cylinder 21A. However, the disclosure is not limited to this. The information detection unit 60 (or the information detection unit 60a) may be provided, e.g., in the vicinity of the spinning pack 16B. Alternatively, information detection units 60 (or information detection units 60a) may be respectively provided in the vicinity of two or more spinning packs 16 (e.g., the spinning packs 16A and 16B). In this case, one information detection unit 60 (or information detection unit 60a) provided in the vicinity of the spinning pack 16A is equivalent to a first detection unit of the present invention. Furthermore, another information detection unit 60 (or another information detection unit 60a) provided in the vicinity of the spinning pack 16B is equivalent to a second detection unit of the present invention. The controller 5 in this case is configured to perform first quality determination of whether the quality of the yarn YA spun out from the spinning pack 16A is stable, based on a detection result by one information detection unit 60 (or one information detection unit 60a) provided in the vicinity of the spinning pack 16A. Furthermore, the controller 5 is configured to perform second quality determination of whether the quality of the yarn YB spun out from the spinning pack 16B is stable, based on a detection result by another information detection unit 60 (or another information detection unit 60a) provided in the vicinity of the spinning pack 16B. Only when both results of the first quality determination and the second quality determination indicate "OK", the controller 5 may cause the spun yarn take-up winder 4 to perform the bobbin switching operation. With this arrangement, the credibility of the quality determination is further increased. Alternatively, information detection units 60 (or information detection units 60a) may be respectively provided in the vicinity of three or more respective spinning packs 16. Alternatively, the information detection unit 60 (or the information detection unit 60a) may be provided in the vicinity of each of all spinning packs 16.
(5) In the embodiment above, the pressure sensor 61 is provided between the gear pumps 15 and the spinning packs 16 in the direction in which molten polymer flows. However, the disclosure is not limited to this. For example, the pressure sensor 61 may be provided at the inside of one spinning pack 16. When such a pressure sensor 61 detects information of the pressure, this information is also equivalent to information regarding the phrase "the pressure of molten polymer flowing into the spinning pack" in the present invention. In this case, when the one spinning pack 16 is replaced, attachment of another spinning pack 16 including a pressure sensor 61 is required.
(6) The embodiment above describes examples in which one result of the quality determination by the controller 5 indicates "OK" immediately after another result of that indicates "NG". In addition to that, as the quality determination, the controller 5 may perform the determination of whether the quality of a yarn Y becomes unstable after the quality of the yarn Y becomes stable. For example, when one or more of the determination parameters do not satisfy the respective standard conditions, the controller 5 resets the number of frequencies of successive OK (or the length of time of successive OK) to zero. When the number of frequencies of successive OK (or the length of time of successive OK) is reset to zero, the controller 5 may immediately cause a result of the quality determination to indicate "NG". Alternatively, after the number of frequencies of successive OK (or the length of time of successive OK) is reset to zero, the controller 5 may cause a result of the quality determination to indicate "OK" until a predetermined time elapses. Furthermore, for example, when the number of frequencies of successive OK is not equal to or larger than the standard frequencies (or when the length of time of successive OK is not equal to or longer than the standard time) after the predetermined time elapses, the controller 5 may cause a result of the quality determination to indicate "NG". With this arrangement, some measures can be taken when the quality of the yarn Y becomes unstable again after the quality of the yarn Y becomes stable. For example, as a measure, the controller 5 may cause the spun yarn take-up winder 4 to perform the bobbin switching operation again when one result of the quality determination indicates "NG" immediately after another result of that indicates "OK". With this arrangement, when the quality of the yarn Y becomes unstable for some reason, one bobbin B onto which the yarn Y is wound is replaced again. It is therefore possible to avoid a case where a low-quality yarn Y is wound onto a high-grade package P.
(7) The controller 5 may perform the determination regarding the grade of each package P which is being formed, based on a result of the quality determination. For example, when one result of the quality determination indicates "NG" immediately after another result of the quality determination indicates "OK", it may be determined that the grade of the package P which is being formed is deteriorated. Based on a result of the quality determination or a result regarding the grade of the package P, the controller 5 may drive the output unit 5b. When a result of the quality determination is different from the last result of the quality determination, the controller 5 may cause the output unit 5b to make an output indicating that the grade of the package P which is being formed varies. With this arrangement, the operator, etc. is notified of information regarding the grade of the package P which is being formed. It is therefore possible to prompt the operator to perform an operation regarding the management of the grade of the package P. Alternatively, instead of the output unit 5b, the controller 5 may send a result of the quality determination to, e.g., a computer device (not illustrated) provided in a central management room (not illustrated) provided for managing the operation state of the spun yarn winding system 1. This computer device may notify the information of an operator in the central management room. In this case, this computer device is equivalent to a notification unit of the present invention. When driving the output unit 5b based on a result of the quality determination or a result of the determination regarding the grade of the package P, the controller 5 may not cause the spun yarn take-up winder 4 to perform the bobbin switching operation. In this case, when the operator makes an input into the input unit 5a, the controller 5 may cause the spun yarn take-up winder 4 to perform the bobbin switching operation in accordance with this input.
(8) In the embodiment above, the controller 5 is configured to control the quality determination and the operation of the spun yarn take-up winder 4. However, the disclosure is not limited to this. For example, the above-described integrated controller (not illustrated) may be configured to acquire information from spinning apparatuses 2 and to control the operation of the spun yarn take-up winder 4 corresponding to each spinning apparatus 2. In this case, the integrated controller is equivalent to the first control unit and second control unit of the present invention.
(9) In the embodiment above, even when a result of the quality determination indicates "NG", the yarn Y is wound onto the bobbin B. However, the disclosure is not limited to this. For example, when a result of the quality determination indicates "NG", the yarn Y spun out from the spinning apparatus 2 may be sucked and removed by an unillustrated suction device before being wound onto the bobbin B by the spun yarn take-up winder 4. In this case, when a result of the quality determination indicates "OK", the operator, etc. may perform yarn threading to the spun yarn take-up winder 4. However, for easy handling of the yarn Y, the yarn Y is preferably wound onto the bobbin B in the same manner as in the embodiment above even when the grade of the yarn Y is low.
(10) In the embodiment above, a combination of the spinning apparatus 2, the cooling apparatus 3, and the controller 5 is equivalent to the yarn spinning system 100. However, the disclosure is not limited to this. A combination of the spinning apparatus 2 and the controller 5 may be handled as the yarn spinning system 100. Alternatively, the yarn spinning system 100 may include other element in addition to the spinning apparatus 2 and the controller 5.
(11) In the embodiment above, the controller 5 is configured to perform the quality determination. However, the disclosure is not limited to this. A computer device (not illustrated) arranged to be able to perform the above-described quality determination may be provided to be independent from the controller 5. In this case, this computer device functions as the first control unit of the present invention. Furthermore, a combination of at least the spinning apparatus 2 (or the spinning apparatus 2a) and this computer device is equivalent to a yarn spinning system of the present invention. Furthermore, the controller 5 may function only as the second control unit of the present invention.

## Claims

1. A yarn spinning system (100, 100a) comprising a spinning apparatus (2, 2a) configured to spin out at least one yarn (Y),
the spinning apparatus (2, 2a) including:
at least one spinning pack (16) including a spinneret (17) configured to discharge molten polymer which is a material of the at least one yarn (Y); and
an information detection unit (60, 60a) arranged to be able to detect information regarding the quality of the at least one yarn (Y) spun out from the spinneret (17),
the yarn spinning system (100, 100a) further comprising a first control unit (5), and
the first control unit (5) being programmed to:
acquire at least one predetermined determination parameter based on a detection result of the information detection unit (60, 60a); and
perform quality determination of whether the quality of the at least one yarn (Y) is stable, with use of the at least one determination parameter;
wherein, the information detection unit (60, 60a) includes at least one of a pressure detection unit (61) which is configured to detect pressure of the molten polymer flowing into the at least one spinning pack (16) and a temperature detection unit (62) which is configured to detect a surface temperature of the spinneret (17);
wherein, the information detection unit (60, 60a) includes the pressure detection unit (61);
**characterized in that** the spinning apparatus (2, 2a) further includes a pushing unit (15) configured to push out the molten polymer toward the at least one spinning pack (16), and
the pressure detection unit (61) is provided between the pushing unit (15) and the at least one spinning pack (16) in a direction in which the molten polymer flows.

2. The yarn spinning system (100, 100a) according to claim 1, wherein, when at least one predetermined standard condition is kept satisfied by the at least one determination parameter for a predetermined standard time, the first control unit (5) determines that the quality of the at least one yarn (Y) is stable.

3. The yarn spinning system (100, 100a) according to claim 2, wherein, the at least one determination parameter includes a detection result parameter regarding a value indicating the detection result,
the at least one standard condition includes a standard detection condition regarding whether the detection result parameter is within a predetermined standard detection range, and
when the detection result parameter is within the standard detection range, the first control unit (5) determines that the detection result parameter satisfies the standard detection condition.

4. The yarn spinning system (100, 100a) according to claim 2 or 3, wherein, the at least one determination parameter includes at least one dispersion parameter regarding dispersion of a value indicating the detection result,
the at least one standard condition includes a standard dispersion condition regarding whether the at least one dispersion parameter is within a predetermined standard dispersion range, and
when the at least one dispersion parameter is within the standard dispersion range, the first control unit (5) determines that the at least one dispersion parameter satisfies the standard dispersion condition.

5. The yarn spinning system (100, 100a) according to claim 4, wherein, the information detection unit (60, 60a) includes the pressure detection unit (61) configured to detect the pressure inside of the at least one spinning pack (16), and
the at least one dispersion parameter includes a variation coefficient of the pressure.

6. A yarn spinning system (100, 100a) comprising a spinning apparatus (2, 2a) configured to spin out at least one yarn (Y),
the spinning apparatus (2, 2a) including:
at least one spinning pack (16) including a spinneret (17) configured to discharge molten polymer which is a material of the at least one yarn (Y); and
an information detection unit (60, 60a) arranged to be able to detect information regarding the quality of the at least one yarn (Y) spun out from the spinneret (17),
the yarn spinning system (100, 100a) further comprising a first control unit (5), and
the first control unit (5) being programmed to:
acquire at least one predetermined determination parameter based on a detection result of the information detection unit (60, 60a); and
perform quality determination of whether the quality of the at least one yarn (Y) is stable, with use of the at least one determination parameter;
wherein, when at least one predetermined standard condition is kept satisfied by the at least one determination parameter for a predetermined standard time, the first control unit (5) determines that the quality of the at least one yarn (Y) is stable;
wherein, the at least one determination parameter includes at least one dispersion parameter regarding dispersion of a value indicating the detection result,
the at least one standard condition includes a standard dispersion condition regarding whether the at least one dispersion parameter is within a predetermined standard dispersion range, and
when the at least one dispersion parameter is within the standard dispersion range, the first control unit (5) determines that the at least one dispersion parameter satisfies the standard dispersion condition;
**characterized in that** the information detection unit (60, 60a) includes the pressure detection unit (61) configured to detect the pressure inside of the at least one spinning pack (16), and
the at least one dispersion parameter includes a variation coefficient of the pressure.

7. The yarn spinning system (100, 100a) according to claim 6, wherein, the spinning apparatus (2, 2a) further includes a spinning beam (14) in which a pack housing (18) in which the at least one spinning pack (16) is detachably housed is formed,
as the spinning pack (16), the pack housing (18) is able to house one spinning pack (16) of one type among spinning packs (16) of plural types for spinning out yarns (Y) that are different from one another in thickness,
the first control unit (5) includes a storage unit (5c) which is able to store information, and
the storage unit (5c) stores information of the standard dispersion range regarding the variation coefficient as information shared between the spinning packs (16) of the plural types.

8. A yarn spinning system (100, 100a) comprising a spinning apparatus (2, 2a) configured to spin out at least one yarn (Y),
the spinning apparatus (2, 2a) including:
at least one spinning pack (16) including a spinneret (17) configured to discharge molten polymer which is a material of the at least one yarn (Y); and
an information detection unit (60, 60a) arranged to be able to detect information regarding the quality of the at least one yarn (Y) spun out from the spinneret (17),
the yarn spinning system (100, 100a) further comprising a first control unit (5), and
the first control unit (5) being programmed to:
acquire at least one predetermined determination parameter based on a detection result of the information detection unit (60, 60a); and
perform quality determination of whether the quality of the at least one yarn (Y) is stable, with use of the at least one determination parameter;
wherein, when at least one predetermined standard condition is kept satisfied by the at least one determination parameter for a predetermined standard time, the first control unit (5) determines that the quality of the at least one yarn (Y) is stable;
**characterized in that** the spinning apparatus (2, 2a) includes the spinning beam (14) in which a pack housing (18) in which the at least one spinning pack (16) is detachably housed is formed,
the pack housing (18) is able to house one spinning pack (16) of one type among spinning packs (16) of plural types for spinning out yarns (Y) that are different from one another in thickness,
the first control unit (5) includes a storage unit (5c) which is able to store information, and
the storage unit (5c) stores information of the standard time as information shared between the spinning packs (16) of the plural types.

9. The yarn spinning system (100, 100a) according to claim 8, wherein, when the at least one determination parameter does not satisfy the at least one standard condition after it is determined that the quality of the at least one yarn (Y) is stable, the first control unit (5) determines that the quality of the at least one yarn (Y) is unstable.

10. The yarn spinning system (100, 100a) according to any one of claims 1 to 9, wherein, the spinning apparatus (2, 2a) includes spinning packs (16),
the spinning packs (16) include a first spinning pack (16A) configured to spin out a first yarn (YA) as the at least one yarn (Y) and a second spinning pack (16B) configured to spin out a second yarn (YB) which is different from the first yarn (YA),
the yarn spinning system (100, 100a) further comprises a cooling apparatus (3) configured to cool the first yarn (YA) and the second yarn (YB) with use of cooling wind,
the cooling apparatus (3) includes:
a first cooling cylinder (21A) which is provided to surround the first yarn (YA) and is configured to allow the cooling wind cooling the first yarn (YA) to pass through the first cooling cylinder (21A);
a second cooling cylinder (21B) which is provided to surround the second yarn (YB) and is configured to allow the cooling wind cooling the second yarn (YB) to pass through the second cooling cylinder (21B); and
a common duct (25) which is provided upstream of the first cooling cylinder (21A) and the second cooling cylinder (21B) in a flow direction in which the cooling wind flows,
the first cooling cylinder (21A) is provided to be close to a downstream end of the common duct (25) in the flow direction as compared to the second cooling cylinder (21B), and
the information detection unit (60, 60a) is provided to correspond to the first spinning pack (16A).

11. The yarn spinning system (100, 100a) according to any one of claims 1 to 9, wherein, the spinning apparatus (2, 2a) includes spinning packs (16),
the spinning packs (16) include a first spinning pack (16A) configured to spin out a first yarn (YA) as the at least one yarn (Y) and a second spinning pack (16B) configured to spin out a second yarn (YB) which is different from the first yarn (YA),
the information detection unit (60, 60a) includes a first detection unit provided to correspond to the first spinning pack (16A) and a second detection unit provided to correspond to the second spinning pack (16B), and
as the quality determination, the first control unit (5) is programmed to perform first quality determination with use of a detection result of the first detection unit and to perform second quality determination with use of a detection result of the second detection unit, the first quality determination being determination of whether the quality of the first yarn (YA) is stable, and the second quality determination being determination of whether the quality of the second yarn (YB) is stable.

12. A spun yarn winding system (1) comprising a yarn spinning system (100, 100a)comprising a spinning apparatus (2, 2a) configured to spin out at least one yarn (Y),
the spinning apparatus (2, 2a) including:
at least one spinning pack (16) including a spinneret (17) configured to discharge molten polymer which is a material of the at least one yarn (Y); and
an information detection unit (60, 60a) arranged to be able to detect information regarding the quality of the at least one yarn (Y) spun out from the spinneret (17),
the yarn spinning system (100, 100a) further comprising a first control unit (5), and
the first control unit (5) being programmed to:
acquire at least one predetermined determination parameter based on a detection result of the information detection unit (60, 60a); and
perform quality determination of whether the quality of the at least one yarn (Y) is stable, with use of the at least one determination parameter;
wherein the spun yarn winding system (1) comprises
a spun yarn take-up winder (4) configured to form a package (P) by winding the at least one yarn (Y); and
a second control unit (5),
the second control unit (5) being programmed to perform, based on a result of the quality determination by the first control unit (5), at least one of control of the spun yarn take-up winder (4) and determination of a grade of the package (P);
**characterized in that** the spun yarn take-up winder (4) includes
a bobbin switching mechanism (47) arranged to be able to perform a bobbin switching operation in which at least one bobbin (B) onto which the at least one yarn (Y) is wound is switched from a predetermined first bobbin (B1) to a second bobbin (B2) which is different from the first bobbin (B1), and
when the first control unit (5) determines that the quality of the at least one yarn (Y) is stable, the second control unit (5) causes the bobbin switching mechanism (47) to perform the bobbin switching operation.

13. The spun yarn winding system (1) according to claim 12, wherein, when the first control unit (5) determines that the quality of the at least one yarn (Y) is unstable again after determining that the quality of the at least one yarn (Y) is stable, the second control unit (5) causes the bobbin switching mechanism (47) to perform the bobbin switching operation again.

14. The spun yarn winding system (1) according to claim 12 or 13, further comprising a notification unit (5b) configured to notify information, wherein,
the second control unit (5) is programmed to drive the notification unit (5b) based on the result of the quality determination by the first control unit (5).

## Patentansprüche

1. Garnspinnsystem (100, 100a), umfassend eine Spinneinrichtung (2, 2a), die ausgebildet ist, mindestens ein Garn (Y) auszuspinnen,
wobei die Spinneinrichtung (2, 2a) Folgendes einschließt:
mindestens ein Spinnpaket (16), das eine Spinndüse (17) einschließt, die ausgebildet ist, geschmolzenes Polymer abzugeben, das ein Material des mindestens einen Garns (Y) ist; und
eine Informationserfassungseinheit (60, 60a), die so angeordnet ist, dass sie Informationen hinsichtlich der Qualität des mindestens einen aus der Spinndüse (17) ausgesponnenen Garns (Y) erfassen kann,
wobei das Garnspinnsystem (100, 100a) weiter eine erste Steuereinheit (5) umfasst, und
wobei die erste Steuereinheit (5) dazu programmiert ist:
mindestens einen vorbestimmten Bestimmungsparameter, basierend auf einem Erfassungsergebnis der Informationserfassungseinheit (60, 60a), zu erfassen; und
eine Qualitätsbestimmung darüber durchzuführen, ob die Qualität des mindestens einen Garns (Y) unter Verwendung des mindestens einen Bestimmungsparameters stabil ist;
wobei die Informationserfassungseinheit (60, 60a) mindestens eine der Druckerfassungseinheit (61), die ausgebildet ist, einen Druck des in das mindestens eine Spinnpaket (16) strömenden geschmolzenen Polymers zu erfassen, und der Temperaturerfassungseinheit (62), die ausgebildet ist, eine Oberflächentemperatur der Spinndüse (17) zu erfassen, einschließt;
wobei die Informationserfassungseinheit (60, 60a) die Druckerfassungseinheit (61) einschließt;
**dadurch gekennzeichnet, dass** die Spinneinrichtung (2, 2a) weiter eine Schubeinheit (15) einschließt, die ausgebildet ist, das geschmolzene Polymer in Richtung des mindestens einen Spinnpakets (16) auszustoßen, und
die Druckerfassungseinheit (61) zwischen der Schubeinheit (15) und dem mindestens einen Spinnpaket (16) in einer Richtung, in der das geschmolzene Polymer strömt, bereitgestellt ist.

2. Garnspinnsystem (100, 100a) nach Anspruch 1, wobei, wenn mindestens eine vorbestimmte Standardbedingung durch den mindestens einen Bestimmungsparameter für eine vorbestimmte Standardzeit erfüllt gehalten wird, die erste Steuereinheit (5) bestimmt, dass die Qualität des mindestens einen Garns (Y) stabil ist.

3. Garnspinnsystem (100, 100a) nach Anspruch 2, wobei der mindestens eine Bestimmungsparameter einen Erfassungsergebnisparameter hinsichtlich eines Werts, der das Erfassungsergebnis angibt, einschließt,
die mindestens eine Standardbedingung eine Standarderfassungsbedingung hinsichtlich dessen einschließt, ob der Erfassungsergebnisparameter innerhalb eines vorbestimmten Standarderfassungsbereichs liegt, und
wenn der Erfassungsergebnisparameter innerhalb des Standarderfassungsbereichs liegt, bestimmt die erste Steuereinheit (5), dass der Erfassungsergebnisparameter die Standarderfassungsbedingung erfüllt.

4. Garnspinnsystem (100, 100a) nach Anspruch 2 oder 3, wobei der mindestens eine Bestimmungsparameter mindestens einen Streuungsparameter hinsichtlich einer Streuung eines Werts, der das Erfassungsergebnis angibt, einschließt,
die mindestens eine Standardbedingung eine Standardstreuungsbedingung hinsichtlich dessen einschließt, ob der mindestens eine Streuungsparameter innerhalb eines vorbestimmten Standardstreuungsbereichs liegt, und
wenn der mindestens eine Streuungsparameter innerhalb des Standardstreuungsbereichs liegt, die erste Steuereinheit (5) bestimmt, dass der mindestens eine Streuungsparameter die Standardstreuungsbedingung erfüllt.

5. Garnspinnsystem (100, 100a) nach Anspruch 4, wobei die Informationserfassungseinheit (60, 60a) die Druckerfassungseinheit (61) einschließt, die ausgebildet ist, den Druck innerhalb des mindestens einen Spinnpakets (16) zu erfassen, und
der mindestens eine Streuungsparameter einen Variationskoeffizienten des Drucks einschließt.

6. Garnspinnsystem (100, 100a), umfassend eine Spinneinrichtung (2, 2a), die ausgebildet ist, mindestens ein Garn (Y) auszuspinnen,
wobei die Spinneinrichtung (2, 2a) Folgendes einschließt:
mindestens ein Spinnpaket (16), das eine Spinndüse (17) einschließt, die ausgebildet ist, geschmolzenes Polymer abzugeben, das ein Material des mindestens einen Garns (Y) ist; und
eine Informationserfassungseinheit (60, 60a), die so angeordnet ist, dass sie Informationen hinsichtlich der Qualität des mindestens einen aus der Spinndüse (17) ausgesponnenen Garns (Y) erfassen kann,
wobei das Garnspinnsystem (100, 100a) weiter eine erste Steuereinheit (5) umfasst, und
wobei die erste Steuereinheit (5) dazu programmiert ist:
mindestens einen vorbestimmten Bestimmungsparameter, basierend auf einem Erfassungsergebnis der Informationserfassungseinheit (60, 60a), zu erfassen; und
eine Qualitätsbestimmung darüber durchzuführen, ob die Qualität des mindestens einen Garns (Y) unter Verwendung des mindestens einen Bestimmungsparameters stabil ist;
wobei, wenn mindestens eine vorbestimmte Standardbedingung durch den mindestens einen Bestimmungsparameter für eine vorbestimmte Standardzeit erfüllt gehalten wird,
die erste Steuereinheit (5) bestimmt, dass die Qualität des mindestens einen Garns (Y) stabil ist;
wobei der mindestens eine Bestimmungsparameter mindestens einen Streuungsparameter hinsichtlich einer Streuung eines Werts, der das Erfassungsergebnis angibt, einschließt,
die mindestens eine Standardbedingung eine Standardstreuungsbedingung hinsichtlich dessen einschließt, ob der mindestens eine Streuungsparameter innerhalb eines vorbestimmten Standardstreuungsbereichs liegt, und
wenn der mindestens eine Streuungsparameter innerhalb des Standardstreuungsbereichs liegt, die erste Steuereinheit (5) bestimmt, dass der mindestens eine Streuungsparameter die Standardstreuungsbedingung erfüllt;
**dadurch gekennzeichnet, dass** die Informationserfassungseinheit (60, 60a) die Druckerfassungseinheit (61) einschließt, die ausgebildet ist, den Druck innerhalb des mindestens einen Spinnpakets (16) zu erfassen, und
der mindestens eine Streuungsparameter einen Variationskoeffizienten des Drucks einschließt.

7. Garnspinnsystem (100, 100a) nach Anspruch 6, wobei die Spinneinrichtung (2, 2a) weiter einen Spinnbalken (14) einschließt, in dem ein Packgehäuse (18) gebildet ist, in dem das mindestens eine Spinnpaket (16) lösbar aufgenommen ist,
hinsichtlich des Spinnpakets (16) kann das Packgehäuse (18) ein Spinnpaket (16) eines Typs unter mehreren Typen von Spinnpaketen (16) zum Ausspinnen von Garnen (Y), die sich voneinander in der Dicke unterscheiden, aufnehmen,
die erste Steuereinheit (5) eine Speichereinheit (5c) einschließt, die in der Lage ist, Informationen zu speichern, und
die Speichereinheit (5c) Informationen des Standardstreuungsbereichs hinsichtlich des Variationskoeffizienten als Informationen speichert, die zwischen den Spinnpaketen (16) der mehreren Typen gemeinsam genutzt werden.

8. Garnspinnsystem (100, 100a), umfassend eine Spinneinrichtung (2, 2a), die ausgebildet ist, mindestens ein Garn (Y) auszuspinnen,
wobei die Spinneinrichtung (2, 2a) Folgendes einschließt:
mindestens ein Spinnpaket (16), das eine Spinndüse (17) einschließt, die ausgebildet ist, geschmolzenes Polymer abzugeben, das ein Material des mindestens einen Garns (Y) ist; und
eine Informationserfassungseinheit (60, 60a), die so angeordnet ist, dass sie Informationen hinsichtlich der Qualität des mindestens einen aus der Spinndüse (17) ausgesponnenen Garns (Y) erfassen kann,
wobei das Garnspinnsystem (100, 100a) weiter eine erste Steuereinheit (5) umfasst, und
wobei die erste Steuereinheit (5) dazu programmiert ist:
mindestens einen vorbestimmten Bestimmungsparameter, basierend auf einem Erfassungsergebnis der Informationserfassungseinheit (60, 60a), zu erfassen; und
eine Qualitätsbestimmung darüber durchzuführen, ob die Qualität des mindestens einen Garns (Y) unter Verwendung des mindestens einen Bestimmungsparameters stabil ist;
wobei, wenn mindestens eine vorbestimmte Standardbedingung durch den mindestens einen Bestimmungsparameter für eine vorbestimmte Standardzeit erfüllt gehalten wird, die erste Steuereinheit (5) bestimmt, dass die Qualität des mindestens einen Garns (Y) stabil ist;
**dadurch gekennzeichnet, dass** die Spinneinrichtung (2, 2a) den Spinnbalken (14) einschließt, in dem ein Packgehäuse (18) gebildet ist, in dem das mindestens eine Spinnpaket (16) lösbar aufgenommen ist,
das Packgehäuse (18) ein Spinnpaket (16) eines Typs unter mehreren Typen von Spinnpaketen (16) zum Ausspinnen von Garnen (Y), die sich voneinander in der Dicke unterscheiden, aufnehmen kann,
die erste Steuereinheit (5) eine Speichereinheit (5c) einschließt, die in der Lage ist, Informationen zu speichern, und
die Speichereinheit (5c) Informationen bezüglich der Standardzeit als Informationen speichert, die zwischen den Spinnpaketen (16) der mehreren Typen gemeinsam genutzt werden.

9. Garnspinnsystem (100, 100a) nach Anspruch 8, wobei, wenn der mindestens eine Bestimmungsparameter die mindestens eine Standardbedingung nicht erfüllt, nachdem bestimmt worden ist, dass die Qualität des mindestens einen Garns (Y) stabil ist, die erste Steuereinheit (5) bestimmt, dass die Qualität des mindestens einen Garns (Y) instabil ist.

10. Garnspinnsystem (100, 100a) nach einem der Ansprüche 1 bis 9, wobei die Spinneinrichtung (2, 2a) Spinnpakete (16) einschließt,
die Spinnpakete (16) ein erstes Spinnpaket (16A) einschließen, das ausgebildet ist, ein erstes Garn (YA) als das mindestens eine Garn (Y) auszuspinnen, und ein zweites Spinnpaket (16B), das ausgebildet ist, ein zweites Garn (YB) auszuspinnen, das sich von dem ersten Garn (YA) unterscheidet,
das Garnspinnsystem (100, 100a) weiter eine Kühleinrichtung (3) umfasst, die ausgebildet ist, das erste Garn (YA) und das zweite Garn (YB) unter Verwendung von Kühlluft zu kühlen,
die Kühleinrichtung (3) Folgendes einschließt:
einen ersten Kühlzylinder (21A), der bereitgestellt ist, um das erste Garn (YA) zu umgeben, und der ausgebildet ist, die das erste Garn (YA) kühlende Kühlluft durch den ersten Kühlzylinder (21A) hindurchtreten zu lassen;
einen zweiten Kühlzylinder (21B), der bereitgestellt ist, um das zweite Garn (YB) zu umgeben, und der ausgebildet ist, die das zweite Garn (YB) kühlende Kühlluft durch den zweiten Kühlzylinder (21B) hindurchtreten zu lassen; und
einen gemeinsamen Kanal (25), der stromaufwärts des ersten Kühlzylinders (21A) und des zweiten Kühlzylinders (21B) in einer Strömungsrichtung, in der die Kühlluft strömt, bereitgestellt ist,
der erste Kühlzylinder (21A) im Vergleich zu dem zweiten Kühlzylinder (21B) so bereitgestellt ist, dass er näher an einem stromabwärtigen Ende des gemeinsamen Kanals (25) in der Strömungsrichtung liegt, und
die Informationserfassungseinheit (60, 60a) dem ersten Spinnpaket (16A) entsprechend bereitgestellt ist.

11. Garnspinnsystem (100, 100a) nach einem der Ansprüche 1 bis 9, wobei die Spinneinrichtung (2, 2a) Spinnpakete (16) einschließt,
die Spinnpakete (16) ein erstes Spinnpaket (16A) einschließen, das ausgebildet ist, ein erstes Garn (YA) als das mindestens eine Garn (Y) auszuspinnen, und ein zweites Spinnpaket (16B), das ausgebildet ist, ein zweites Garn (YB) auszuspinnen, das sich von dem ersten Garn (YA) unterscheidet,
die Informationserfassungseinheit (60, 60a) eine erste Erfassungseinheit einschließt, die dem ersten Spinnpaket (16A) entsprechend bereitgestellt ist, und eine zweite Erfassungseinheit, die dem zweiten Spinnpaket (16B) entsprechend bereitgestellt ist, und
die erste Steuereinheit (5) für die Qualitätsbestimmung so programmiert ist, dass sie eine erste Qualitätsbestimmung unter Verwendung eines Erfassungsergebnisses der ersten Erfassungseinheit und eine zweite Qualitätsbestimmung unter Verwendung eines Erfassungsergebnisses der zweiten Erfassungseinheit durchführt, wobei die erste Qualitätsbestimmung eine Bestimmung dessen ist, ob die Qualität des ersten Garns (YA) stabil ist, und die zweite Qualitätsbestimmung eine Bestimmung dessen ist, ob die Qualität des zweiten Garns (YB) stabil ist.

12. Spinngarnwickelsystem (1), umfassend ein Garnspinnsystem (100, 100a), umfassend eine Spinneinrichtung (2, 2a), die ausgebildet ist, mindestens ein Garn (Y) auszuspinnen,
wobei die Spinneinrichtung (2, 2a) Folgendes einschließt:
mindestens ein Spinnpaket (16), das eine Spinndüse (17) einschließt, die ausgebildet ist, geschmolzenes Polymer abzugeben, das ein Material des mindestens einen Garns (Y) ist; und
eine Informationserfassungseinheit (60, 60a), die so angeordnet ist, dass sie Informationen hinsichtlich der Qualität des mindestens einen aus der Spinndüse (17) ausgesponnenen Garns (Y) erfassen kann,
wobei das Garnspinnsystem (100, 100a) weiter eine erste Steuereinheit (5) umfasst, und
wobei die erste Steuereinheit (5) dazu programmiert ist:
mindestens einen vorbestimmten Bestimmungsparameter, basierend auf einem Erfassungsergebnis der Informationserfassungseinheit (60, 60a), zu erfassen; und
eine Qualitätsbestimmung darüber durchzuführen, ob die Qualität des mindestens einen Garns (Y) unter Verwendung des mindestens einen Bestimmungsparameters stabil ist;
wobei das Spinngarnwickelsystem (1) Folgendes umfasst
einen Spinngarn-Aufnahme-Aufwickler (4), der ausgebildet ist, durch Aufwickeln des mindestens einen Garns (Y) ein Paket (P) zu bilden; und
eine zweite Steuereinheit (5),
die zweite Steuereinheit (5) so programmiert ist, dass sie, basierend auf einem Ergebnis der Qualitätsbestimmung durch die erste Steuereinheit (5), mindestens eine der Regelung des Spinngarn-Aufnahme-Aufwicklers (4) und der Bestimmung eines Qualitätsgrads des Pakets (P) durchführt;
**dadurch gekennzeichnet, dass** der Spinngarn-Aufnahme-Aufwickler (4) Folgendes einschließt:
einen Spulenwechselmechanismus (47), der so angeordnet ist, dass er einen Spulenwechselvorgang durchführen kann, bei dem mindestens eine Spule (B), auf die das mindestens eine Garn (Y) gewickelt ist, von einer vorbestimmten ersten Spule (B1) auf eine zweite Spule (B2), die sich von der ersten Spule (B1) unterscheidet, gewechselt wird, und
wenn die erste Steuereinheit (5) bestimmt, dass die Qualität des mindestens einen Garns (Y) stabil ist, veranlasst die zweite Steuereinheit (5) den Spulenwechselmechanismus (47), den Spulenwechselvorgang durchzuführen.

13. Spinngarnwickelsystem (1) nach Anspruch 12, wobei, wenn die erste Steuereinheit (5) bestimmt, dass die Qualität des mindestens einen Garns (Y) wieder instabil ist, nachdem bestimmt worden ist, dass die Qualität des mindestens einen Garns (Y) stabil ist, die zweite Steuereinheit (5) den Spulenwechselmechanismus (47) veranlasst, den Spulenwechselvorgang erneut durchzuführen.

14. Spinngarnwickelsystem (1) nach Anspruch 12 oder 13, das weiter eine Meldeeinheit (5b) umfasst, die ausgebildet ist, Informationen zu melden, wobei,
die zweite Steuereinheit (5) so programmiert ist, dass sie die Meldeeinheit (5b), basierend auf dem Ergebnis der Qualitätsbestimmung durch die erste Steuereinheit (5), ansteuert.

## Revendications

1. Système de filature de fil (100, 100a) comprenant un appareil de filature (2, 2a) configuré pour filer au moins un fil (Y),
l'appareil de filature (2, 2a) incluant :
au moins un pack de filature (16) incluant une filière (17) configurée pour évacuer du polymère fondu qui est un matériau du au moins un fil (Y) ; et
une unité de détection d'informations (60, 60a) conçue pour pouvoir détecter des informations concernant la qualité du au moins un fil (Y) filé à partir de la filière (17),
le système de filature de fil (100, 100a) comprenant en outre une première unité de commande (5), et
la première unité de commande (5) étant programmée pour :
acquérir au moins un paramètre de détermination prédéterminé sur la base d'un résultat de détection de l'unité de détection d'informations (60, 60a) ; et
effectuer une détermination de qualité quant à savoir si la qualité du au moins un fil (Y) est stable, en utilisant le au moins un paramètre de détermination ;
dans lequel, l'unité de détection d'informations (60, 60a) inclut au moins une parmi une unité de détection de pression (61) qui est configurée pour détecter la pression du polymère fondu entrant dans le au moins un pack de filature (16) et une unité de détection de température (62) qui est configurée pour détecter une température de surface de la filière (17) ;
dans lequel, l'unité de détection d'informations (60, 60a) inclut l'unité de détection de pression (61) ;
**caractérisé en ce que** l'appareil de filature (2, 2a) inclut en outre une unité de poussée (15) configurée pour pousser le polymère fondu vers le au moins un pack de filature (16), et
l'unité de détection de pression (61) est disposée entre l'unité de poussée (15) et le au moins un pack de filature (16) dans une direction dans laquelle le polymère fondu s'écoule.

2. Système de filature de fil (100, 100a) selon la revendication 1, dans lequel, lorsqu'au moins une condition standard prédéterminée reste satisfaite par le au moins un paramètre de détermination pendant un temps standard prédéterminé, la première unité de commande (5) détermine que la qualité du au moins un fil (Y) est stable.

3. Système de filature de fil (100, 100a) selon la revendication 2, dans lequel, le au moins un paramètre de détermination inclut un paramètre de résultat de détection concernant une valeur indiquant le résultat de détection,
la au moins une condition standard inclut une condition de détection standard quant à savoir si le paramètre de résultat de détection est dans une plage de détection standard prédéterminée, et
lorsque le paramètre de résultat de détection est dans la plage de détection standard, la première unité de commande (5) détermine que le paramètre de résultat de détection satisfait à la condition de détection standard.

4. Système de filature de fil (100, 100a) selon la revendication 2 ou la revendication 3, dans lequel, le au moins un paramètre de détermination inclut au moins un paramètre de dispersion concernant la dispersion d'une valeur indiquant le résultat de détection,
la au moins une condition standard inclut une condition de dispersion standard précisant si le au moins un paramètre de dispersion est dans une plage de dispersion standard prédéterminée, et
lorsque le au moins un paramètre de dispersion est dans la plage de dispersion standard, la première unité de commande (5) détermine que le au moins un paramètre de dispersion satisfait à la condition de dispersion standard.

5. Système de filature de fil (100, 100a) selon la revendication 4, dans lequel, l'unité de détection d'informations (60, 60a) inclut l'unité de détection de pression (61) configurée pour détecter la pression à l'intérieur du au moins un pack de filature (16), et
le au moins un paramètre de dispersion inclut un coefficient de variation de la pression.

6. Système de filature de fil (100, 100a) comprenant un appareil de filature (2, 2a) configuré pour filer au moins un fil (Y),
l'appareil de filature (2, 2a) incluant :
au moins un pack de filature (16) incluant une filière (17) configurée pour évacuer du polymère fondu qui est un matériau du au moins un fil (Y) ; et
une unité de détection d'informations (60, 60a) conçue pour pouvoir détecter des informations concernant la qualité du au moins un fil (Y) filé à partir de la filière (17),
le système de filature de fil (100, 100a) comprenant en outre une première unité de commande (5), et
la première unité de commande (5) étant programmée pour :
acquérir au moins un paramètre de détermination prédéterminé sur la base d'un résultat de détection de l'unité de détection d'informations (60, 60a) ; et
effectuer une détermination de qualité quant à savoir si la qualité du au moins un fil (Y) est stable, en utilisant le au moins un paramètre de détermination ;
dans lequel, lorsqu'au moins une condition standard prédéterminée reste satisfaite par le au moins un paramètre de détermination pendant un temps standard prédéterminé, la première unité de commande (5) détermine que la qualité du au moins un fil (Y) est stable ;
dans lequel, le au moins un paramètre de détermination inclut au moins un paramètre de dispersion concernant la dispersion d'une valeur indiquant le résultat de détection,
la au moins une condition standard inclut une condition de dispersion standard précisant si le au moins un paramètre de dispersion est dans une plage de dispersion standard prédéterminée, et
lorsque le au moins un paramètre de dispersion est dans la plage de dispersion standard, la première unité de commande (5) détermine que le au moins un paramètre de dispersion satisfait à la condition de dispersion standard ;
**caractérisé en ce que** l'unité de détection d'informations (60, 60a) inclut l'unité de détection de pression (61) configurée pour détecter la pression à l'intérieur du au moins un pack de filature (16), et
le au moins un paramètre de dispersion inclut un coefficient de variation de la pression.

7. Système de filature de fil (100, 100a) selon la revendication 6, dans lequel, l'appareil de filature (2, 2a) inclut en outre un bloc de filature (14) dans lequel est formé un logement de pack (18) dans lequel le au moins un pack de filature (16) est logé de manière amovible,
en tant que pack de filature (16), le logement de pack (18) peut loger un pack de filature (16) d'un type parmi une pluralité de types de packs de filature (16) destinés à filer des fils (Y) d'épaisseurs différentes,
la première unité de commande (5) inclut une unité de stockage (5c) qui peut stocker des informations, et
l'unité de stockage (5c) stocke des informations concernant la plage de dispersion standard relative au coefficient de variation en tant qu'informations partagées entre les packs de filature (16) de la pluralité de types.

8. Système de filature de fil (100, 100a) comprenant un appareil de filature (2, 2a) configuré pour filer au moins un fil (Y),
l'appareil de filature (2, 2a) incluant :
au moins un pack de filature (16) incluant une filière (17) configurée pour évacuer du polymère fondu qui est un matériau du au moins un fil (Y) ; et
une unité de détection d'informations (60, 60a) conçue pour pouvoir détecter des informations concernant la qualité du au moins un fil (Y) filé à partir de la filière (17),
le système de filature de fil (100, 100a) comprenant en outre une première unité de commande (5), et
la première unité de commande (5) étant programmée pour :
acquérir au moins un paramètre de détermination prédéterminé sur la base d'un résultat de détection de l'unité de détection d'informations (60, 60a) ; et
effectuer une détermination de qualité quant à savoir si la qualité du au moins un fil (Y) est stable, en utilisant le au moins un paramètre de détermination ;
dans lequel, lorsqu'au moins une condition standard prédéterminée reste satisfaite par au moins un paramètre de détermination pendant un temps standard prédéterminé, la première unité de commande (5) détermine que la qualité d'au moins un fil (Y) est stable ;
**caractérisé en ce que** l'appareil de filature (2, 2a) inclut le bloc de filature (14) dans lequel est formé un logement de pack (18) dans lequel le au moins un pack de filature (16) est logé de manière amovible,
le logement de pack (18) peut loger un pack de filature (16) d'un type parmi une pluralité de types de packs de filature (16) destinés à filer des fils (Y) d'épaisseurs différentes,
la première unité de commande (5) inclut une unité de stockage (5c) qui peut stocker des informations, et
l'unité de stockage (5c) stocke des informations concernant le temps standard en tant qu'informations partagées entre les packs de filature (16) de la pluralité de types.

9. Système de filature de fil (100, 100a) selon la revendication 8, dans lequel, lorsque le au moins un paramètre de détermination ne satisfait pas à la au moins une condition standard après que la qualité du au moins un fil (Y) a été déterminée comme stable, la première unité de commande (5) détermine que la qualité du au moins un fil (Y) est instable.

10. Système de filature de fil (100, 100a) selon l'une quelconque des revendications 1 à 9, dans lequel, l'appareil de filature (2, 2a) inclut des packs de filature (16),
les packs de filature (16) incluent un premier pack de filature (16A) configuré pour filer un premier fil (YA) en tant que le au moins un fil (Y) et un deuxième pack de filature (16B) configuré pour filer un deuxième fil (YB) qui est différent du premier fil (YA),
le système de filature de fil (100, 100a) comprend en outre un appareil de refroidissement (3) configuré pour refroidir le premier fil (YA) et le deuxième fil (YB) en utilisant de l'air de refroidissement,
l'appareil de refroidissement (3) inclut :
un premier cylindre de refroidissement (21A) qui est disposé de manière à entourer le premier fil (YA) et est configuré pour laisser l'air de refroidissement refroidissant le premier fil (YA) passer à travers le premier cylindre de refroidissement (21A) ;
un deuxième cylindre de refroidissement (21B) qui est disposé de manière à entourer le deuxième fil (YB) et est configuré pour laisser l'air de refroidissement refroidissant le deuxième fil (YB) passer à travers le deuxième cylindre de refroidissement (21B) ; et
un conduit commun (25) qui est disposé en amont du premier cylindre de refroidissement (21A) et du deuxième cylindre de refroidissement (21B) dans une direction d'écoulement dans laquelle l'air de refroidissement s'écoule,
le premier cylindre de refroidissement (21A) est disposé de manière à être proche d'une extrémité aval du conduit commun (25) dans la direction d'écoulement par comparaison avec le deuxième cylindre de refroidissement (21B), et
l'unité de détection d'informations (60, 60a) est disposée de manière à correspondre au premier pack de filature (16A).

11. Système de filature de fil (100, 100a) selon l'une quelconque des revendications 1 à 9, dans lequel, l'appareil de filature (2, 2a) inclut des packs de filature (16),
les packs de filature (16) incluent un premier pack de filature (16A) configuré pour filer un premier fil (YA) en tant que le au moins un fil (Y) et un deuxième pack de filature (16B) configuré pour filer un deuxième fil (YB) qui est différent du premier fil (YA),
l'unité de détection d'informations (60, 60a) inclut une première unité de détection disposée de manière à correspondre au premier pack de filature (16A) et une deuxième unité de détection disposée de manière à correspondre au deuxième pack de filature (16B), et
en tant que détermination de qualité, la première unité de commande (5) est programmée pour effectuer une première détermination de qualité en utilisant un résultat de détection de la première unité de détection et pour effectuer une deuxième détermination de qualité en utilisant un résultat de détection de la deuxième unité de détection, la première détermination de qualité déterminant si la qualité du premier fil (YA) est stable, et la deuxième détermination de qualité déterminant si la qualité du deuxième fil (YB) est stable.

12. Système d'enroulement de fil filé (1) comprenant un système de filature de fil (100, 100a) comprenant un appareil de filature (2, 2a) configuré pour filer au moins un fil (Y),
l'appareil de filature (2, 2a) incluant :
au moins un pack de filature (16) incluant une filière (17) configurée pour évacuer du polymère fondu qui est un matériau du au moins un fil (Y) ; et
une unité de détection d'informations (60, 60a) conçue pour pouvoir détecter des informations concernant la qualité du au moins un fil (Y) filé à partir de la filière (17),
le système de filature de fil (100, 100a) comprenant en outre une première unité de commande (5), et
la première unité de commande (5) étant programmée pour :
acquérir au moins un paramètre de détermination prédéterminé sur la base d'un résultat de détection de l'unité de détection d'informations (60, 60a) ; et
effectuer une détermination de qualité quant à savoir si la qualité du au moins un fil (Y) est stable, en utilisant le au moins un paramètre de détermination ;
dans lequel le système d'enroulement de fil filé (1) comprend
un enrouleur de reprise de fil filé (4) configuré pour former un paquet (P) par enroulement du au moins un fil (Y) ; et
une deuxième unité de commande (5),
la deuxième unité de commande (5) étant programmée pour effectuer, sur la base d'un résultat de la détermination de qualité par la première unité de commande (5), au moins l'une parmi la commande de l'enrouleur de reprise de fil filé (4) et la détermination d'une classe du paquet (P) ;
**caractérisé en ce que** l'enrouleur de reprise de fil filé (4) inclut
un mécanisme de commutation de bobines (47) conçu pour pouvoir effectuer une opération de commutation de bobines dans laquelle au moins une bobine (B) sur laquelle au moins un fil (Y) est enroulé passe d'une première bobine prédéterminée (B1) à une deuxième bobine (B2) qui est différente de la première bobine (B1), et
lorsque la première unité de commande (5) détermine que la qualité du au moins un fil (Y) est stable, la deuxième unité de commande (5) amène le mécanisme de commutation de bobines (47) à effectuer l'opération de commutation de bobines.

13. Système d'enroulement de fil filé (1) selon la revendication 12, dans lequel, lorsque la première unité de commande (5) détermine que la qualité du au moins un fil (Y) est de nouveau instable après avoir déterminé que la qualité du au moins un fil (Y) est stable, la deuxième unité de commande (5) amène le mécanisme de commutation de bobines (47) à effectuer de nouveau l'opération de commutation de bobines.

14. Système d'enroulement de fil filé (1) selon la revendication 12 ou la revendication 13, comprenant en outre une unité de notification (5b) configurée pour notifier des informations, dans lequel,
la deuxième unité de commande (5) est programmée pour entraîner l'unité de notification (5b) sur la base du résultat de la détermination de qualité par la première unité de commande (5).
